# EUROPÄISCHE PATENTSCHRIFT

(11) **EP 1 867 325 B1**
(45) Veröffentlichungstag und Bekanntmachung des Hinweises auf die Patenterteilung: **14.09.2011**
(21) Anmeldenummer: 06019183.0
(22) Anmeldetag: 19.03.1999
(51) Int. Cl.: A61K 9/50, B01J 13/02

(54) **Kapseln enthaltend Lipide in der Hülle**
Capsules comprising lipids in the coating
Capsules comprenant des lipides dans le revêtement

(30) Priorität: 19.03.1998 DE 19812083; 15.07.1998 EP 98113181; 22.02.1999 DE 19907552
(43) Veröffentlichungstag der Anmeldung: 19.12.2007
(62) Teilanmeldung aus: 05027659.1
(73) Patentinhaber: Max-Planck-Gesellschaft zur Förderung der Wissenschaften e.V., 80539 München (DE)
(72) Erfinder: Donath, Edwin, Prof. Dr., 04316 Leipzig (DE); Sukhorukov, Gleb, Dr., Moscow 115280 (RU); Lerche, Karl-Heinz, Dr., 10243 Berlin (DE); Voigt, Andreas, Dr., 12621 Berlin (DE); Bäumler, Hans, Dr., 12437 Berlin (DE); Caruso, Frank, Prof. Dr., Preston Victoria 3072 (AU); Möhwald, Helmuth, Prof. Dr., 55411 Bingen (DE)
(74) Vertreter: Vossius & Partner

(56) Entgegenhaltungen:
- DE-A1- 4 312 970
- GB-A- 2 145 992
- US-A- 4 663 286
- US-A- 5 091 187
- US-A- 5 487 390
- SUKHORUKOV G B ET AL: "LAYER-BY-LAYER SELF ASSEMBLY OF POLYELECTROLYTES ON COLLOIDAL PARTICLES" COLLOIDS AND SURFACES. A, PHYSICACHEMICAL AND ENGINEERING ASPECTS, ELSEVIER, AMSTERDAM, NL, Bd. 137, 1. Januar 1998 (1998-01-01), Seiten 253-266, XP000852906 ISSN: 0927-7757
- SUKHORUKOV G B ET AL: "STPEWISE POLYELECTROLYTE ASSEMBLY ON PARTICLE SURFACES: A NOVEL APPROACH TO COLLOID DESIGN" POLYMERS FOR ADVANCED TECHNOLOGIES, WILEY & SONS, BOGNOR REGIS, GB, Bd. 9, Nr. 10/11, 1. Oktober 1998 (1998-10-01), Seiten 759-767, XP000784433 ISSN: 1042-7147
- CARUSO F ET AL: "INFLUENCE OF POLYELECTROLYTE MULTILAYER COATINGS ON FOERSTER RESONANCE ENERGY TRANSFER BETWEEN 6-CARBOXYFLUORESCEIN AND RHODAMINE B-LABELED PARTICLES IN AQUEOUS SOLUTION" JOURNAL OF PHYSICAL CHEMISTRY. B, MATERIALS, SURFACES, INTERFACES AND BIOPHYSICAL, WASHINGTON, DC, US, Bd. 102, 24. Februar 1998 (1998-02-24), Seiten 2011-2016, XP000852731 ISSN: 1089-5647

## Beschreibung

Die Erfindung betrifft Nano- bzw. Mikrokapseln, die eine Polyelektrolythülle umfassen, ein Verfahren zur Herstellung dieser Kapseln sowie ihre Verwendung.

Mikrokapseln sind in verschiedenen Ausführungsformen bekannt und werden insbesondere für die kontrollierte Freisetzung und den zielgerichteten Transport von pharmazeutischen Wirkstoffen sowie zum Schutz von empfindlichen Wirkstoffen, wie etwa Enzymen und Proteinen verwendet (siehe z.B. D.D.Lewis, "Biodegradable Polymers and Drug Delivery Systems", M.Chasin and R.Langer, Hrsg. (Marcel Decker, New York, 1990); J.P.McGee et al., J.Control.Release 34 (1995), 77).

Mikrokapseln können durch mechanisch-physikalische Verfahren, wie z.B. Versprühen und nachfolgende Beschichtung hergestellt werden. Die auf diese Weise erhältlichen Mikrokapseln weisen jedoch eine Reihe von Nachteilen auf. Insbesondere ist es mit den bekannten mechanisch-physikalischen Verfahren nicht möglich, Mikrokapseln mit einer Größe von < 10 µm (Durchmesser) herzustellen. Vielmehr sind nur Mikrokapseln mit relativ großen Durchmessern erhältlich, deren Anwendungsbereich jedoch aufgrund ihrer Größe beschränkt ist. Weiterhin wird bei den bekannten mechanisch-physikalischen Verfahren keine monodisperse Kapselverteilung, sondern vielmehr eine uneinheitliche Verteilung von Kapseln verschiedener Größe erhalten. Auch dies ist für viele Anwendungen, bei denen die Größe der Kapsel wichtig ist, nachteilig.

Neben den mechanisch-physikalischen Verfahren sind auch chemische Verfahren zur Herstellung von Mikrokapseln bekannt. So können Mikrokapseln durch Grenzflächenpolymerisation bzw. -kondensation oder durch Polymerphasentrennung aus einem Polymer/Lösungsmittelgemisch hergestellt werden (B.Miksa et al., Colloid Polym.Sci.273 (1995), 47; G.Crotts et al., J.Control.Release 35 (1995), 91; S.L.Regen et al., J.Am.Chem.Soc.106 (1984), 5756). Aber auch die durch bekannte chemische Verfahren hergestellten Mikrokapseln weisen eine Reihe von Nachteilen auf. Insbesondere sind eine hohe Polydispersität, eine ungleichmäßige Umhüllung sowie oftmals eine Verfestigung des Kerns zu beobachten. Ein weiterer wesentlicher Nachteil der bekannten chemischen Verfahren liegt in der Verwendung von organischen Lösungsmitteln und polymerisierbaren organischen Monomeren, was zu beträchtlichen Beschränkungen hinsichtlich der verwendbaren einzukapselnden Wirkstoffe führt. Insbesondere die oftmals dadurch notwendige Verwendung von mit Wasser nicht mischbaren organischen Flüssigkeiten als Kernmaterial schränkt den Anwendungsbereich solcher Mikrokapseln, gerade im Hinblick auf Proteine oder Enzyme drastisch ein.

Ein weiteres System, das zur Einkapselung von anorganischen und organischen Materialien verwendet wurde, sind Lipidliposomen (D.D.Lasic, "Liposomes: From Physics to Applications" (Elsevier, Amsterdam, 1993); S.L.Regen et al., J.Am.Chem.Soc.106 (1984), 2446). Die Einkapselung von Wirkstoffen in Lipidliposomen gestattet die Herstellung von Mikrokapseln unter relativ schonenden Bedingungen, weshalb Liposomen als Trägersysteme für verschiedene pharmazeutische und kosmetische Wirkstoffe verwendet werden. Die biologische, chemische und mechanische Stabilität solcher Liposomkapseln ist jedoch sehr gering, was die allgemeine Verwendbarkeit solcher Kapseln limitiert. Einen weiteren Nachteil stellt die geringe Permeabilität von Liposomenkapseln, insbesondere für polare Moleküle, dar, die einen Stoffaustausch mit dem Umgebungsmedium verhindert.

Bei einem weiteren Verfahren zur Herstellung von Mikrokapseln werden zunächst Mischungen aus dem einzuschließenden Material und einem mit z.B. Ca²⁺-lonen verfestigbaren Polyelektrolytbestandteil gebildet. Diese Mischung wird in Form kleinster Tröpfchen in ein Ca²⁺-Bad eingebracht, wobei sich eine Gelstruktur bildet, die dann in weiteren Verfahrensschritten mit einer Polyelektrolytkapsel umgeben werden kann. Eine Weiterentwicklung solcher Verfahren wird in DE 33 06 259 A1 beschrieben, wobei auf die Verwendung von Ca²⁺ verzichtet werden kann. Der hauptsächliche Nachteil dieser Verfahren besteht darin, daß die Größe der herstellbaren Mikrokapseln nach unten auf etwa 50 µm (Durchmesser) begrenzt ist und die Wandstärke der erhaltenen Mikrokapseln mindestens 100 nm beträgt.

In DE-A-40 26 978 ist ein Verfahren zur Beschichtung von flächigen Trägern beschrieben, wobei ein Träger so modifiziert wird, daß er flächenweit Ionen oder ionisierbare Verbindungen mit gleichsinniger Ladung trägt und eine oder mehrere Schichten aus organischen Materialien, die in jeder Schicht gleichsinnig geladene Ionen enthalten, aus einer Lösung solcher organischer Materialien auf den modifizierten Träger aufgetragen wird, wobei das organische Material für die erste Schicht Ionen mit entgegengesetztem Ladungsinn zum Ladungssinn der lonenmodifizierung des Trägers aufweist und im Falle von mehreren Schichten abwechselnd weitere Schichten mit Ionen mit dem jeweils entgegengesetzten Ladungssinn zur vorhergehenden in gleicher Weise wie die erste Schicht aufgetragen werden. Als Träger werden anorganische oder organische Trägermaterialien mit ebenmäßiger Oberfläche offenbart. Es findet sich keinerlei Hinweis auf die Verwendung von Mikropartikeln als Trägermaterialien oder auf eine Auflösung der Trägermaterialien nach der Beschichtung.

EP 1 098 696 B1 betrifft ein Verfahren zur Herstellung von Kapseln mit einer Polyelektrolythülle, dadurch gekennzeichnet, dass man auf einem Templat ausgewählt aus Aggregaten biologischer oder/und amphiphiler Materialien mehrere aufeinanderfolgende Schichten entgegengesetzt geladener Polyelektrolytmoleküle aufbringt, indem man zunächst eine Dispersion der Templatpartikel in einer wässrigen Lösung erzeugt, zu dieser Dispersion dann eine Polyelektrolytspezies mit der gleichen oder der entgegengesetzten Ladung wie die Oberfläche des Templatpartikels gibt, anschließend nach Abtrennung gegebenenfalls vorhandener überschüssiger Polyelektrolytmoleküle die für den Aufbau der zweiten Schicht verwendete entgegengesetzt geladene Polyelektrolytspezies zugibt und dann gegebenenfalls weitere abwechselnd entgegengesetzt geladene Schichten von Polyelektrolytmolekülen aufgebracht werden und anschließend gegebenenfalls das Templat desintegriert.

EP 1 647 326 betrifft ein Verfahren zur Herstellung von Kapseln mit einer Polyelektrolythülle, die mehrere Polyelektrolytschichten umfasst, und einen Durchmesser bis zu 10 µm aufweist, umfassend die Schritte:
a) Bereitstellen einer wässrigen Dispersion von Templatpartikeln geeigneter Größe,
b) Herstellen einer Hülle um die Partikel durch Aufbringen von Polyelektrolyten auf die Templatpartikel, und
c) zumindest teilweise Desintegration der Templatpartikel.

EP 1 064 087 B1 betrifft Kapseln mit einer Polyelektrolythülle, die mehrere Polyelektrolytschichten umfasst, und einen Durchmesser bis zu 10 µm aufweist, dadurch gekennzeichnet, dass die Hülle abwechselnde Schichten von kationischen und anionischen Polyelektrolyten umfasst, und dass die Kapsel einen Wirkstoff ausgewählt aus pharmazeutischen Wirkstoffen, Katalysatoren, Sensormolekülen, Pflanzenschutzmitteln und Aromastoffen enthält.

Eine Aufgabe der Erfindung bestand deshalb darin, Kapseln mit einem geringen Durchmesser bereitzustellen, in denen Materialen, wie etwa Makromoleküle, Präzipitate, Flüssigkeiten oder Gase, eingeschlossen werden können. Die Kapseln sollten weiterhin eine hohe Stabilität und Hüllen mit geringer Wandstärke aufweisen, die insbesondere für Ionen und kleine Moleküle durchlässig sind.

Die Aufgabe wird erfindungsgemäß gelöst durch Kapseln mit einer Polyelektrolythülle, die mehrere Polyelektrolytschichten umfasst, und einem Durchmesser bis zu 10 µm, dadurch gekennzeichnet, dass die Hülle Lipide enthält.

Überraschenderweise wurde festgestellt, daß bei Beschichtung von Templatpartikeln mit einer Polyelektrolythülle und gegebenenfalls anschließender Desintegration der Templatpartikel Kapseln mit definierten inneren und äußeren Hülleigenschaften und mit selektiv steuerbaren Permeabilitätseigenschaften erhalten werden können. Unter einer Polyelektrolythülle wird eine Hülle mit einem Gehalt an Polyelektrolyten verstanden. Bevorzugt besteht die Polyelektrolythülle zu mindestens 50% insbesondere mindestens 60% und besonders bevorzugt zu mindestens 80% aus Polyelektrolyten. Die erfindungsgemäßen Kapseln gestatten den Einschluß auch empfindlicher Moleküle unter schonenden Bedingungen, z.B. in wäßrigen Lösungen. Die Kapselwand ist eine Polyelektrolythülle, die den Stoffaustausch im Hinblick auf niedermolekulare Stoffe und Ionen mit der Umgebung ermöglicht, aber gleichzeitig makromolekulare Stoffe zurückhält. Diese Trennfunktion der Polyelektrolythülle bewirkt einerseits, daß gegebenfalls in die Kapsel eingeschlossene Wirkstoffe zurückgehalten werden, andererseits, daß von außen keine störenden makromolekularen Stoffe in die Kapsel gelangen können. Auf diese Weise werden Wirkstoffe auch ohne den Zusatz von konservierenden Stoffen effektiv vor biologischen Abbauprozessen geschützt. Die chemischen und physikalischen Eigenschaften der als Kapselwand dienenden Polyelektrolythülle können in weiten Grenzen durch den Aufbau und die Zusammensetzung der Hülle sowie durch Umgebungsparameter gesteuert werden. So können die erfindungsgemäßen Kapseln beispielsweise als Transporträume dienen, wobei hier die Parameter der äußeren Schicht den Transport an vorgegebene Zielorte, z.B. im Organismus, bestimmen.

Die erfindungsgemäßen Kapseln umfassen Mikrokapseln mit einem Durchmesser von ≤ 10 µm, besonders bevorzugt ≤5 µm und am meisten bevorzugt ≤ 2 µm sowie Nanokapseln mit einem Durchmesser von ≥ 10 nm bis < 1000 nm.

Die Hülle der Kapseln weist mehrere Polyelektrolytschichten auf. Unter Polyelektrolyten werden allgemein Polymere mit ionisch dissoziierbaren Gruppen, die Bestandteil oder Substituent der Polymerkette sein können, verstanden. Üblicherweise ist die Zahl dieser ionisch dissoziierbaren Gruppen in Polyelektrolyten so groß, daß die Polymeren in der dissoziierten Form (auch Polyionen genannt) wasserlöslich sind. Hierin werden unter dem Begriff Polyelektrolyte auch Ionomere verstanden, bei denen die Konzentration der ionischen Gruppen für eine Wasserlöslichkeit nicht ausreichend sind, die jedoch genügend Ladungen aufweisen, um eine Selbstassemblierung einzugehen. Bevorzugt umfasst die Hülle "echte" Polyelektrolyte. Je nach Art der dissoziierbaren Gruppen werden Polyelektrolyte in Polysäuren und Polybasen unterteilt. Aus Polysäuren entstehen bei der Dissoziation unter Abspaltung von Protonen Polyanionen, die sowohl anorganische als auch organische Polymere sein können. Beispiele für Polysäuren sind Polyphosphorsäure, Polyvinylschwefelsäure, Polyvinyl-sulfonsäure, Polyvinylphosphonsäure und Polyacrylsäure. Beispiele für die entsprechenden Salze, die auch als Polysalze bezeichnet werden, sind Polyphosphat, Polysulfat, Polysulfonat, Polyphosphonat und Polyacrylat.

Polybasen enthalten Gruppen, die in der Lage sind, Protonen, z.B. durch Reaktion mit Säuren unter Salzbildung, aufzunehmen. Beispiele für Polybasen mit ketten- bzw. seitenständigen dissoziierbaren Gruppen sind Polyethylenimin, Polyvinylamin und Polyvinylpyridin. Polybasen bilden durch Aufnahme von Protonen Polykationen.

Erfindungsgemäß geeignete Polyelektrolyte sind sowohl Biopolymere, wie etwa Alginsäure, Gummi arabicum, Nucleinsäuren, Pektine, Proteine und andere, sowie chemisch modifizierte Biopolymere, wie etwa ionische oder ionisierbare Polysaccharide, z.B. Carboxymethylcellulose, Chitosan und Chitosansulfat, Ligninsulfonate sowie synthetische Polymere, wie etwa Polymethacrylsäure, Polyvinylsulfonsäure, Polyvinylphosphonsäure und Polyethylenimin.

Es können lineare oder verzweigte Polyelektrolyte eingesetzt werden. Die Verwendung verzweigter Polyelektrolyte führt zu weniger kompakten Polyelektrolytmultifilmen mit einem höheren Grad der Wandporosität. Zur Erhöhung der Kapselstabilität können Polyelektrolytmoleküle innerhalb oder/und zwischen den einzelnen Schichten vernetzt werden, z.B. durch Crosslinking von Aminogruppen mit Aldehyden. Weiterhin können amphiphile Polyelektrolyte, z.B. amphiphile Block- oder Randomcopolymere mit partiellem Polyelektrolytcharakter zur Verringering der Permeabilität gegenüber polaren kleinen Molekülen eingesetzt werden. Solche amphiphilen Copolymere bestehen aus Einheiten unterschiedlicher Funktionalität, z.B. einerseits sauren oder basischen Einheiten und andererseits hydrophoben Einheiten wie Styrolen, Dienen oder Siloxanen etc. die als Blöcke oder statistisch verteilt über das Polymer angeordnet sein können. Durch Verwendung von Copolymeren, die als Funktion äußerer Bedingungen ihre Struktur ändern, können die Kapselwände bezüglich ihrer Permeabilität oder anderer Eigenschaften definiert gesteuert werden. Hierzu bieten sich beispielsweise Copolymere mit einem Poly(N-isopropylacrylamid)-Anteil, z.B. Poly(N-isopropylacrylamid-acrylsäure) an, die über das Gleichgewicht von Wasserstoffbrückenbindungen ihre Wasserlöslichkeit als Funktion der Temperatur ändern, was mit einer Quellung einhergeht.

Durch Verwendung von unter bestimmten Bedingungen abbaubaren, z.B. photo-, säure-, base- oder salzlabilen Polyelektrolyten kann über die Auflösung der Kapselwände die Freisetzung von eingeschlossenen Wirkstoffen gesteuert werden. Weiterhin können für bestimmte Anwendungsmöglichkeiten auch leitende Polyelektrolyten oder Polyelektrolyten mit optisch aktiven Gruppen als Kapselkomponenten verwendet werden.

Durch geeignete Wahl der Polyelektrolyte ist es möglich, die Eigenschaften und Zusammensetzung der Polyelektrolythülle der erfindungsgemäßen Kapseln definiert einzustellen. Insbesondere bei schichtweise aufgebauten Polyelektrolythüllen kann die Zusammensetzung der Hüllen durch die Wahl der Substanzen beim Schichtaufbau in weiten Grenzen variiert werden. Grundsätzlich ergeben sich keine Einschränkungen hinsichtlich der zu verwendenden Polyelektrolyte bzw. Ionomere, solange die verwendeten Moleküle eine genügend hohe Ladung aufweisen oder/und die Fähigkeit besitzen, über andere Wechselwirkungsarten, wie beispielsweise Wasserstoffbrückenbindungen und/oder hydrophobe Wechselwirkungen, eine Bindung mit der darunter liegenden Schicht einzugehen.

Geeignete Polyelektrolyte sind somit sowohl niedermolekulare Polyelektrolyte bzw. Polyionen als auch makromolekulare Polyelektrolyte, beispielsweise Polyelektrolyte biologischer Herkunft.

Von besonderer Bedeutung für die Verwendung der Kapseln ist die Permeabilität der Hüllwand. Wie bereits oben ausgeführt, ermöglicht die Vielzahl der zur Verfügung stehenden Polyelektrolyte die Herstellung einer Vielzahl von Hüllkompositionen mit unterschiedlichen Eigenschaften. Insbesondere kann die elektrische Ladung der Außenhülle dem Anwendungszweck angepaßt werden. Zudem kann die Innenhülle an jeweils verkapselte Wirkstoffe angepaßt werden, wodurch z.B. eine Stabilisierung des Wirkstoffs erzielt werden kann. Daneben kann auch die Permeabilität der Hüllwand durch die Wahl der Polyelektrolyte in der Hülle und durch die Wanddicke sowie die Umgebungsbedingungen beeinflußt werden. Dadurch ist eine selektive Gestaltung der Permeabilistätseigenschaften sowie eine definierte Veränderung dieser Eigenschaften möglich.

Die Permeabilitätseigenschaften der Hülle können durch Poren in mindestens einer der Polyelektrolytschichten weiter modifiziert werden. Solche Poren können bei geeigneter Wahl durch die Polyelektrolyten selbst gebildet werden. Neben den Polyelektrolyten kann die Hülle aber auch andere Substanzen umfassen, um eine gewünschte Permeabilität zu erzielen. So kann insbesondere durch Einbringen von Nanopartikeln mit anionischen oder/und kationischen Gruppen oder von grenzflächenaktiven Substanzen, wie etwa Tensiden oder/und Lipiden, die Permeabilität für polare Komponenten gesenkt werden. Erfindungsgemäß wird die Permeabilität von Polyelektrolythüllen dadurch modifiziert, dass auf der Polyelektrolythülle Lipidschichten abgeschieden werden. Durch die Inkorporation von selektiven Transportsystemen, wie z.B. Carriern oder Kanälen, in die Polyelektrolythülle, insbesondere in Lipidschichten, ist eine genaue Anpassung der transversalen Transporteigenschaften der Hülle an den jeweiligen Anwendungszweck möglich. Die Poren oder Kanäle der Hüllwand können durch chemische Modifizierung oder/und Änderung der Umgebungsbedingungen gezielt geöffnet bzw. verschlossen werden. So führt beispielsweise eine hohe Salzkonzentration des Umgebungsmediums zu einer hohen Durchlässigkeit der Hüllwand.

Eine besonders bevorzugte Modifizierung der Permeabilität von Polyelektrolythüllen kann durch Abscheidung von Lipidschichten oder/und amphiphiler Polyelektrolyten auf der Polyelektrolythülle nach Desintegration der Templatpartikel erreicht werden. Auf diese Weise kann die Permeabilität der Polyelektrolythüllen für kleine und polare Moleküle sehr stark vermindert werden. Beispiele für Lipide, die auf den Polyelektrolythüllen abgeschieden werden können, sind Lipide, die mindestens eine ionische oder ionisierbare Gruppe tragen, z.B. Phospholipide wie etwa Dipalmitoylphosphatidinsäure oder zwitterionische Phospholipide wie etwa Dipalmitoylphosphatidylcholin oder auch Fettsäuren bzw. entsprechende langkettige Alkylsulfonsäuren. Bei Verwendung zwitterionischer Lipide können Lipidmultischichten auf der Polyelektrolythülle abgeschieden werden. Auf den Lipidschichten können anschließend weitere Polyelektrolytschichten abgeschieden werden.

Die erfindungsgemäßen Kapseln weisen bevorzugt eine Hüllwanddicke von 2 bis 1000 nm, insbesondere 2 bis 100 nm, z.B. von 5 bis 8 nm, auf. Die Dicke der Hüllwand ist abhängig von der Anzahl der Schichten, der Polyelektrolythülle. Die Kapseln enthalten bevorzugt 2 bis 40, bevorzugt 2 bis 20, z.B. 3 bis 10 Schichten. Die Kapseln können jedoch auch eine größere Anzahl von Schichten, d.h. Polyelektrolytschichten und gegebenenfalls andere Schichten wie Lipidschichten, enthalten.

Die erfindungsgemäßen Kapseln zeichnen sich weiterhin durch ihre Monodispersität aus. So ist es möglich, eine Zusammensetzung mit einer Kapselverteilung zu erhalten, bei der der Anteil an Kapseln, deren Abweichung vom mittleren Durchmesser > 50% ist, weniger als 20%, bevorzugt weniger als 10% und besonders bevorzugt weniger als 1 %.

Die Kapseln sind sehr stabil gegenüber chemischen, biologischen, mechanischen und thermischen Belastungen. Die Kapseln können gegebenenfalls mit eingeschlossenen Wirkstoffen ohne Beeinträchtigung ihrer Eigenschaften getrocknet, eingefroren oder/und gefriergetrocknet werden. Nach dem Auftauen bzw. Resuspendieren in Wasser werden wieder intakte Kapseln erhalten.

Bei Trocknung oder Gefriertrocknung der Kapseln wird eine pulverförmige Zusammensetzung erhalten, die in geeigneten Lösungsmitteln, insbesondere in wäßrigen Lösungen resuspendiert werden kann. Ein weiterer Gegenstand der Erfindung ist deshalb eine getrocknete Kapseln umfassende Zusammensetzung. Die Trocknung kann nach bekannten Verfahren durchgeführt werden, insbesondere bei erhöhter oder verringerter Temperatur oder/und reduziertem Druck.

Weiterhin wird ein Verfahren zur Herstellung von mit einer Polyelektrolythülle beschichteten Kapseln betrachtet, umfassend die Schritte:
a) Bereitstellen einer wäßrigen Dispersion von Templatpartikeln geeigneter Größe und
b) Herstellen einer Hülle um die Templatpartikel durch Aufbringen von Polyelektrolyten auf die Templatpartikel.

Zunächst wird eine wäßrige Dispersion von Templatpartikeln geeigneter Größe bereitgestellt. Durch die Größe der Templatpartikel wird die Größe der Kapseln festgelegt. Anschließend werden vorzugsweise mehrere Polyelektrolytschichten auf die Templatpartikel aufgebracht, wodurch ein umhülltes Templatpartikel gebildet wird. Die Form der Hülle hängt dabei unmittelbar von der Form der Templatpartikel ab.

Zum Aufbringen der Polyelektrolytschichten auf das Templat wird vorzugsweise eine Dispersion der Templatpartikel in einer wässrigen Lösung erzeugt. Zu dieser Dispersion gibt man dann Polyelektrolytmoleküle, aus denen die erste Schicht aufgebaut werden soll. Diese Polyelektrolytmoleküle können die gleiche oder die entgegengesetzte Ladung wie die Oberfläche der Templatpartikel haben. Die Menge der zugegebenen Polyelektrolytmoleküle wird so gewählt, daß das gesamte Material für den Aufbau der ersten Schicht benötigt wird oder es wird ein Überschuß verwendet. Im letzteren Fall ist eine Entfernung der überschüssigen Polyelektrolytmoleküle, die nicht zum Aufbau der ersten Schicht benötigt werden, zweckmäßig, bevor die Zugabe von entgegengesetzt geladenen Polyelektrolytmolekülen für den Aufbau der zweiten Schicht erfolgt. Die Abtrennung der Polyelektrolytmoleküle kann nach bekannten Verfahren erfolgen, insbesondere Zentrifugation, Filtration oder/und Dialyse. Besonders bevorzugt ist die Abtrennung durch Membranfiltration wie im folgenden beschrieben. Anschließend werden weiterhin abwechselnd entgegengesetzt geladene Schichten von Polyelektrolytmolekülen aufgebracht, wobei für jede Schicht mit gleicher Ladung gleiche oder verschiedene Polyelektrolytspezies oder Gemische von Polyelektrolytspezies gewählt werden können. Die Anzahl von Schichten kann grundsätzlich beliebig gewählt werden. Neben Polyelektrolytmolekülen können auch andere Substanzen wie Nanopartikel, oberflächenaktive Substanzen oder/und Lipide auf den Templatpartikeln abgeschieden werden.

Ein weiterer Gegenstand der Erfindung ist ein Verfahren zur Modifizierung der Permeabilität von Polyelektrolythüllen, dadurch gekennzeichnet, dass auf der Polyelektrolythülle Lipidschichten abgeschieden werden.

Als Templatpartikel können sowohl anorganische Materialien, z.B. Metalle, Keramiken, Oxide oder Salzkristalle als auch organische Materialien wie Polymerlatizes oder Melaminformaldehydpartikel, Lipidvesikel oder biologische Templatpartikel eingesetzt werden. Ebenfalls geeignet sind Emulsionströpfchen. Die Größe der Templatpartikel kann - insbesondere bei Verwendung biologischer Templatmaterialien - bis zu 10 µm erreichen, besonders bevorzugt 5 nm bis 5 µm. Die Form der Templatpartikel ist nicht kritisch. Sowohl sphärische als auch anisotrope Partikel können beschichtet werden.

Es können auch Aggregate von Subpartikeln als Ausgangskerne (Templatpartikel) zur Beschichtung mit Polyelektrolyten eingesetzt werden. Diese Aggregate können gegebenenfalls in vorgeformtem oder präformiertem Zustand eingesetzt werden. Eine solche Präformierung kann beispielsweise durch Anlegen externer elektrischer Gleichstrom- oder/und Wechselfelder bzw. Magnetfelder auf Suspensionen mit Subpartikeln erzielt werden. Durch vorgeformte Aggregate kann die Form der Kapseln bestimmt werden. Weiterhin können solche Aggregate mit einer hohen Einheitlichkeit hinsichtlich der Größenverteilung erhalten werden (Monodispersität). Ebenso geeignet sind jedoch auch nichtvorgeformte Aggregate. Von besonderem Interesse sind Aggregate in sphärischer Form.

Die verwendeten Templatpartikel müssen nicht notwendigerweise geladen sein, um eine Selbstassemblierung von Polyelektrolytschichten zu ermöglichen. Vielmehr kann auf nichtgeladene Kerne ein geladener Precursorfilm aufgebracht werden, der an die Templatpartikel durch andere Wechselwirkungen, beispielsweise hydrophobe Wechselwirkungen, gebunden ist.

Nach Aufbringen der gewünschten Anzahl von Polyelektrolytschichten können die umhüllten Templatpartikel - sofern gewünscht - desintegriert, insbesondere zerkleinert oder aufgelöst werden. Dabei verbleiben "leere" Kapseln mit einer Polyelektrolythülle. Die Auflösung der Templatpartikel wird unter Bedingungen durchgeführt, bei denen die Hüllen intakt bleiben. Eine Auflösung kann je nach Wahl des Templatpartikelmaterials z.B. thermisch oder chemisch erfolgen. Die bei der Auflösung entstehenden niedermolekularen Kernbestandteile können durch die Poren der Hülle nach außen gelangen. Auf diese Weise erhält man Kapseln mit Polyelektrolythüllen, die einen "leeren" Kern enthalten. Auf die leeren Polyelektrolytmolekülen können anderen Beschichtungssubstanzen aufgebracht werden.

Nach Desintegration der Templatpartikel kann im Inneren der Kapselhülle eine Flüssigphase vorliegen. Grundsätzlich können die Kapseln jede Flüssigkeit in ihrem Innern enthalten, z.B. eine wäßrige Flüssigkeit, insbesondere eine wäßrige Salzlösung oder Wasser, oder auch organische Lösungsmittel, insbesondere mit Wasser nicht mischbare Lösungsmittel wie Alkohole oder Kohlenwasserstoffe mit mindestens 4 C-Atomen. Weiterhin können die Kapseln in ihrem Inneren auch Feststoffe oder Gase enthalten.

Bevorzugt werden teilvernetzte Melaminformaldehydpartikel als auflösbare Templatpartikel eingesetzt, die durch Einstellung des pH-Werts in dem die umhüllten Partikel enthaltenden Medium auf einen sauren Wert, z.B. ≤ 1,5, aufgelöst werden können, während die Hüllschicht selbst intakt bleibt. Die teilvernetzten Melaminformaldehydpartikel können auch durch chemische Reaktionen, insbesondere durch Sulfonierung in wäßrigen Medien aufgelöst werden. Als Sulfonierungsagentien werden bevorzugt Alkalisulfate, Alkalihydrogensulfite und andere wasserlösliche Salze der schwefeligen Säure verwendet. Weitere Beispiele für auflösbare Templatpartikel sind lösliche Polymerkerne, z.B. Harnstoff-Formaldehydpartikel, oder Salzkristalle.

Außerdem können als Templatmaterialien beispielsweise Zellen, z.B. eukaryontische Zellen, wie etwa Säugererythrozyten oder Pflanzenzellen, einzellige Organismen wie etwa Hefen, Bakterienzellen wie etwa E.coli Zellen, Zellaggregate, subzelluläre Partikel wie etwa Zellorganelle, Pollen, Membranpräparationen oder Zellkerne, Viruspartikel und Aggregate von Biomolekülen, z.B. Proteinaggregate wie etwa Immunkomplexe, kondensierte Nukleinsäuren, Ligand-Rezeptor-Komplexe etc. verwendet werden. Das erfindungsgemäße Verfahren eignet sich auch zur Verkapselung lebender biologischer Zellen und Organismen. Ebenso als Template geeignet sind Aggregate amphiphiler Materialien, insbesonder Membranstrukturen wie etwa Vesikel, z.B. Liposomen oder Micellen sowie andere Lipidaggregate.

Die Desintegration biologischer Templatpartikel kann durch Zugabe von Lysereagenzien erfolgen. Dabei sind Lysereagenzien geeignet, die biologische Materialien wie Proteine oder/und Lipide auflösen können. Vorzugsweise enthalten die Lysereagenzien ein Deproteinisierungsmittel, beispielsweise Peroxoverbindungen wie etwa H₂O₂ oder/und Hypochloritverbindungen wie etwa Natrium- oder Kaliumhypochlorit. Überraschenderweise erfolgt die Desintegration der Templatpartikel innerhalb einer kurzen Inkubationsdauer, z.B. 1 min bis 1 h bei Raumtemperatur. Die Desintegration der Templatpartikel ist weitgehend vollständig, da selbst bei elektronenmikroskopischer Betrachtung der verbleibenden Hüllen keine Reste der Partikel mehr nachweisbar sind. Bei Einbau biologischer Polyelektrolyte in die Hülle können leere Schichten auch innerhalb der Polyelektrolythülle erzeugt werden.

Die bei der Desintegration der Templatpartikel gebildeten Fragmente, z.B. im Fall von teilvernetzten Melaminformaledhypartikeln, die bei Auflösung entstandenen Oligomere, können durch Poren, insbesondere Nanoporen, der Hüllwand aus dem Inneren der Kapseln nach außen austreten. Anschließend können sie - sofern gewünscht - von den Kapseln abgetrennt werden. Diese Abtrennung kann durch dem Fachmann bekannte Verfahren durchgeführt werden, z.B. durch Dialyse, Filtration oder/und Zentrifugation abgetrennt. Eine Abtrennung von Templatpartikelfragmenten ist oftmals jedoch nicht notwendig. Die Kapseln können auch ohne Abtrennungsschritt verwendet werden.

Mit dem erfindungsgemäßen Verfahren ist es auch möglich, Kapseln mit eingeschlossenen Wirkstoffen oder Kapseln zum Einschluß von Wirkstoffen herzustellen. Die Beladung des Innenraums mit kleinen Molekülen kann dadurch erfolgen, daß die Permeabilität der Hülle als Funktion der externen physikalischen und chemischen Parameter variiert wird. Zur Beladung wird ein Zustand hoher Permeabilität eingestellt. Das eingeschlossene Material wird anschließend durch Veränderung der äußeren Parameter oder/und Verschluß der Poren, beispielsweise durch Kondensation der Hülle oder chemische Modifikation der Poren oder Kanäle zurückgehalten.

Die Wirkstoffe können sowohl anorganische als auch organische Stoffe sein. Beispiele für solche Wirkstoffe sind Katalysatoren, insbesondere Enzyme, Nanopartikel, pharmazeutische Wirkstoffe, Polymere, Farbstoffe wie etwa fluoreszierende Verbindungen, Sensormoleküle, d.h. Moleküle, die auf die Änderung von Umgebungsbedingungen (Temperatur, pH-Wert) nachweisbar reagieren, Pflanzenschutzmittel und Aromastoffe. Da die Kapseln in ihrem Kern wäßrige Lösungen enthalten können, ist es möglich, daß auch empfindliche Moleküle unter schonenden Bedingungen eingeschlossen werden.

Bei Einschluß von Katalysatoren, z.B. keramischen oder/und metallischen Partikeln oder Enzymen, in die Kapseln können die Katalysatoren entweder an der Innenseite der Kapselwand adsorbiert sein oder als freie Moleküle im Kapselinnenraum vorliegen, so daß eine nahezu verlustfreie Verwendung der Katalysatoren ermöglicht wird. Die Katalysator enthaltenden Kapseln können einfacher als der freie Katalysator zurückgehalten oder rückgewonnen werden. Eine Kontamination der Katalysatoren wird durch die Schutz- und Trennfunktion der Kapselhülle gegenüber dem Umgebungsmedium weitgehend ausgeschlossen. Insbesondere wird durch die Permeabilitätseigenschaften der Kapselwände verhindert, daß die im Kapselinneren eingeschlossenen Katalysatoren durch makromolekulare Stoffe in ihrer Wirksamkeit gehemmt oder inhibiert werden, während der Zutritt von Substrat und Austritt von Produkten möglich ist.

Die Kapseln können auch eingeschlossene pharmazeutische Wirkstoffe enthalten. In diesem Fall wirkt die Kapsel insbesondere als Transportvehikel, um die pharmazeutischen Wirkstoffe zu stabilisieren, vor einem Abbau zu schützen oder/und an den gewünschten Wirkungsort im Organimus zu transportieren. Durch Auswahl der Oberflächeneigenschaften der äußeren Hülle kann ein spezifischer Transport erzielt werden.

Die Polyelektrolythülle der Kapseln ist vorzugsweise für niedermolekulare Substanzen durchlässig, verhindert aber den Durchtritt von Makromolekülen. Damit stellt die Hüllwand eine Barriere gegenüber Mikroorganismen und von ihnen sekretierten externen Verdauungsenzymen dar. Deshalb können in die erfindungsgemäßen Kapseln biologisch abbaubare Substanzen eingeschlossen sein, ohne daß Konservierungsstoffe zur Haltbarmachung notwendig wären.

Die Kapseln können auch als Reaktionsräume für chemische Reaktionen oder als Präzipitations- oder Kristallisationstemplate verwendet werden, wobei man leere Kapseln oder einen Wirkstoff oder Katalysator enthaltende Kapseln einsetzen kann. Aufgrund der Tatsache, daß die Permeabilität der Kapselwände steuerbar ist, so daß sie beispielsweise niedermolekulare Substanzen passieren lassen, Makromoleküle jedoch weitgehend zurückhalten, lassen sich bei einer chemischen Reaktion entstehende hochmolekulare Produkte, z.B. bei einer Polymerisation entstehende Polymere auf einfache Weise während der Synthese im Innenraum zurückhalten. Das gleichzeitig im Außenmedium synthetisierte Reaktionsprodukt kann z.B. durch Zentrifugation oder/und Filtration nachträglich oder auch bereits während der Reaktion entfernt werden.

Während der Reaktion kann die Zufuhr des Reaktionssubstrats über die Diffusion durch die Kapselwände gesteuert werden. Dabei ergeben sich neue Wege, in Reaktionsabläufe einzugreifen. Da z.B. durch Filtration ein kontinuierlicher oder z.B. durch Zentrifugation auch ein plötzlicher Austausch des Außenmediums möglich ist, kann die Polymerisationsreaktion durch Substratentfernung beliebig angehalten werden bzw. das Monomer kann ausgetauscht werden. Es ist somit möglich, auf neue Art und Weise die Herstellung von definierten Co- oder Multipolymeren durchzuführen. Da durch die Permeation der Reaktionsablauf über die Monomerenzufuhr kontrollierbar ist, können in den Kapseln Produkte mit neuen und anderen Molekulargewichtsverteilungen, z.B. hoch monodisperse Produkte erzeugt werden. Im Kapselinneren synthetisierte Polymere lassen sich z.B. durch NMR, durch IR, spektroskopisch durch Titration mit Fluoreszenzfarbstoffen und durch konfokale Mikroskopie nachweisen. Mit Einzelteilchenlichtstreuung läßt sich der Massenzuwachs und somit die Reaktionskinetik verfolgen.

Bei Verwendung anisotroper Kapseln zur Verpackung von Wirkstoffen oder als Reaktionsräume, z.B. für Synthesen oder Präzipitationsprozesse, und gegebenenfalls anschließender Auflösung der Templathüllen können Partikelzusammensetzungen als Dispersionen mit vorbestimmten Formen und Gestalten erzeugt werden. Die Erfindung betrifft somit auch anisotrope Partikelzusammensetzungen, die durch Verkapselung von Wirkstoffen in einer Polyelektrolythülle, z.B. durch Synthese oder Präzipitation und anschließende Entfernung des Templats, z.B. durch thermische oder chemische Behandlung, erhältlich sind. Vorzugsweise besitzen diese anisotropen Partikel die Form der als Templat verwendeten Strukturen. Anisotrope Partikel können bei Anlagen von Felder bewegt, z.B. gedreht oder ausgerichtet werden. Auf diese Weise können Dispersionen mit schaltenden Eigenschaften erzeugt werden.

Weiterhin können die Kapseln zum Einbringen von organischen Flüssigkeiten wie etwa Alkoholen oder Kohlenwasserstoffen, z.B. Hexanol, Octanol, Octan oder Decan, oder zum Verkapseln von Gasen verwendet werden. Solche mit einer organischen, nicht mit Wasser mischbaren Flüssigkeit gefüllten Kapseln können auch für chemische Reaktionen, z.B. Polymerisationsreaktionen eingesetzt werden. So kann das Monomer über dessen Verteilungsgleichgewicht gezielt im Innenraum der Kapseln angereichert werden. Gegebenenfalls kann die Monomerenlösung bereits vor Beginn der Synthese im Innenraum eingekapselt werden.

Es können jedoch auch Wirkstoffe verkapselt werden, die aufgrund ihrer Größe nicht die Polyelektrolythülle durchdringen können. Hierzu wird der einzuschließende Wirkstoff an das Templatpartikel gekoppelt bzw. immobilisiert oder vom Templatpartikel eingekapselt oder aufgenommen, z.B. durch Phagozytose oder Endozytose bei lebenden Zellen oder durch Verkapselung von Nanopartikeln in lösliche Templatmaterialien. Nach Desintegration der Templatpartikel wird der Wirkstoff ins Innere der Polyelektrolythülle freigesetzt. Dabei werden zweckmäßigerweise die Bedingungen bei der Desintegration des Templatpartikels so gewählt, daß keine unerwünschte Zersetzung des Wirkstoffs eintritt.

Eine Kopplung des Wirkstoffs an das Templat kann direkt erfolgen, aber auch durch einen Bindevermittler bewirkt werden. Als Bindevermittler werden bevorzugt Moleküle verwendet, die bei bestimmten Bedingungen degradierbar oder abbaubar sind. Besonders bevorzugt wird als Bindevermittler Polymilchsäure verwendet. Hierzu wird der Wirkstoff mittels des Bindevermittlers, insbesondere Polymilchsäure, an das Templatpartikel, beispielsweise ein teilvernetztes Melaminformaldehydpartikel immobilisiert. Auf diese Weise wird der einzuschließende Wirkstoff selbst Bestandteil des Schichtaufbaus bei der Beschichtung des Kerns. Nach der Auflösung der Templatpartikel und ggf. Degradation der Bindemoleküle wird der Wirkstoff ins Innere der Hülle freigesetzt. Mit diesem Verfahren können beliebige Wirkstoffe in die Hülle eingeschlossen werden, insbesondere Nanopartikel und nichtbiologische makromolekulare Komponenten und bevorzugt biologische Makromoleküle, wie etwa Proteine, insbesondere Enzyme.

Weiterhin können z.B. mit 4-Pyrensulfat (4-PS) kationische Polymere oder Partikel in der Hülle fixiert werden. Durch Herauslösen von 4-PS in Salzlösungen werden diese Partikel dann in das Innere der Hülle freigesetzt.

Die Inkorporation von Wirkstoffen in den von den Hüllen umschlossenen Innenraum kann aber auch durch vorherige Einbringung der Wirkstoffe in die Templatpartikel bei Verwendung von reversiblen Mikrogelen als Templatpartikel durchgeführt werden. So ermöglicht beispielsweise die Verwendung von teilvernetzten Methylolmelaminkernen vor der Beschichtung, in gequollene Kerne Substanzen zu inkorporieren, die nach einer reversiblen Schrumpfung im Kern eingeschlossen sind.

Die Kapseln können auch auf einer Oberfläche immobilisiert werden. Die Einstellung der Ladung der äußeren Schicht und die freie Funktionalisierbarkeit der Außenhülle erlaubt eine vom Zustand der eingeschlossenen Moleküle unabhängige Immobilisierung der Kapseln. Dies eröffnet zahlreiche Anwendungsmöglichkeiten, vor allem im Bereich der Sensorik und Oberflächenanalytik. Dabei können die mit Polyelektrolyt beschichteten Templatpartikel an eine Oberfläche adheriert und die Templatpartikel dann aus den bereits immobilisierten beschichteten Kernen herausgelöst werden, um immobilisierte Kapseln zu bilden. Ebenso kann jedoch die Auflösung der Kerne vor einer Deponierung an der Oberfläche erfolgen.

Die Kapseln können auf zahlreichen Anwendungsgebieten, beispielsweise Sensorik, Oberflächenanalytik, als Emulsionsträger, Mikroreaktionsräume wie etwa für katalytische Prozesse, Polymerisation-, Präzipitations- oder Kristallisationsprozesse, in der Pharmazie und Medizin, z.B. zum Targeting von Wirkstoffen oder als Ultraschallkontrastmittel, in der Lebensmitteltechnologie, Kosmetik, Biotechnologie, Sensorik, Informationstechnologie, Druckindustrie (Einkapselung von Farbstoffen), photographische Industrie und für Tiermedizin oder Landwirtschaft (Wirkstoffe für Tiergesundheit, Wirkstoffe für Landwirtschaft oder Gartenbau) eingesetzt werden. Weiterhin können die Kapseln zum Aufbau von Mikro- bzw. Nanokompositen, d.h. Werkstoffen, die aus mindestens zwei verschiedenen Materialien bestehen und eine mikro- bzw. nanoskopische Ordnung aufweisen, eingesetzt werden.

Bei der Verwendung der Kapseln als Reaktionsräume können niedermolekulare Substanzen, wie z.B. Edukte und Produkte, durch die Hüllwände permeiren, während beispielsweise die Katalysatoren eingeschlossen sind. Bei der Verwendung von mit Katalysatoren beladenen Mikro- bzw. Nanokapseln, wobei die Kapseln beispielsweise in einer Säule gepackt sind, steht für die Reaktion beträchtlich mehr Katalysator zur Verfügung, als bei herkömmlichen oberflächengebundenen Katalysatoren, da dort die Größe der Oberfläche limitierend ist. Besonders vorteilhaft ist, daß der Katalysator im Kapselinneren von der Produktion nicht durch aufwendige Verfahren wieder abgetrennt werden muß. Weiterhin ist die Lebensdauer der Katalysatoren verbessert, da makromolekulare Stoffe, insbesondere Bakterien und Pilze, nicht durch die Hüllwände gelangen können. Dadurch werden die an viele Verfahren gestellten hohen Anforderungen an die Sterilität verringert, was viele, technisch einfache Anwendungen von biologischen Katalysatoren eröffnet.

Auch Sensormoleküle können in die Kapseln eingeschlossen werden. Dabei kann es sich um Enzyme handeln, die in Anwesenheit eines Substrats unter geeigneten Bedingungen optisch oder auf andere Weise nachweisbare Produkte, beispielsweise farbige oder fluoreszierende Produkte bilden. Es können aber auch elektrisch aktive Sensormoleküle, insbesondere oxidierbare oder reduzierbare Stoffe, eingeschlossen werden, wobei die Kapseln auf Elektroden immobilisiert werden können. Hier ist neben der schützenden Funktion der Kapseln insbesondere von Vorteil, daß das Sensormolekül, nicht direkt mit der Elektrode in Berührung kommt.

Die Kapseln können auch zur Herstellung von Kristallen oder amorphen Präzipitaten aus organischen oder anorganischen Materialien oder zum Einschluß von organischen oder anorganischen Kristallen oder amorphen Präzipitaten verwendet werden. Bevorzugt dienen die Kapseln als Kristallisations- oder Präzipitationsraum bzw. Template zur Herstellung von insbesondere monodispersen Kristallen oder Präzipitaten. Mit den erfindungsgemäßen Kapseln ist ein hoher Grad an Monodispersität zu erhalten, da die maximale Größe der eingeschlossenen Partikel durch die Größe der Kapseln begrenzt ist. Als Kristallisationskeime können chemische Gruppen an der inneren Hüllwand verwendet werden. Dazu werden beim schichtweisen Aufbau der Hülle der Kapseln in der innersten Schicht Moleküle verwendet, die Seitenketten aufweisen, die das Kristallwachstum begünstigen. So können beispielsweise Polyphosphate an der Innenseite der Hülle aufgebracht werden, um im Inneren CaCO₃ zu bilden. Als äußerste Schicht der Polyelektrolythülle der Kapseln werden günstigerweise Polyelektrolyte verwendet, die ein Kristallwachstum unterdrücken, beispielsweise Amine.

Die Kapseln können auch zum Aufbau von Mikro- bzw. Nanokompositen verwendet werden. Mikro- und Nanokomposite sind Werkstoffe, die aus mindestens zwei verschiedenen Materialien bestehen und eine mikro- bzw. nanoskopische Ordnung aufweisen. Solche Komposite imitieren oftmals in der Natur vorliegende Produkte, wie z.B. Muschelschalen, die aus Nanokompositen von gewöhnlichen Kalk- und Eiweißmolekülen bestehen. Solche Komposite haben bei geringem Gewicht eine überraschend hohe Festigkeit. Durch die Assemblierung können makroskopische Strukturen geordnet aufgebaut werden.

Anisotrope Hüllen, die unter Verwendung anisotroper Templatpartikel, z.B. biologischer Templatpartikel hergestellt wurden, erlauben in Verbindung mit z.B. Kristallisation oder/und Präzipitation die Herstellung von Kompositen mit anisotropen Eigenschaften. So können beispielsweise magnetische Ellipsoide hergestellt werden, z.B. durch Füllung mit magnetischen Partikeln oder/und durch Adsorption von magnetischen Nanopartikeln auf der Polyelektrolythülle. Im Magnetfeld zeigen diese anisotropen Partikel eine Orientierung, wodurch die optischen Eigenschaften einer Partikelsuspension rasant geändert werden können (magneto-optischer Schalter). Auf analoge Weise kann man mit ferroelektrischen Partikeln verfahren. Mit Hilfe dieser Teilchen kann man beispielsweise mit einem rotierenden Feld kleine Schaufelräder zum Pumpen erzeugen (Mikromechanik). Daneben können anisotrope Partikel durch Dissipation erwärmt werden. Dies kann zur Erzeugung extrem lokalisierter Wärmequellen genutzt werden, die man mit elektrischen bzw. mit magnetischen Feldern bewegen kann. Dies erlaubt die Erzeugung lokaler Hyperthermieffekte. Weiterhin können durch geordnete Ausrichtung anisotroper Partikel Kompositmaterialien mit hierarchischer Struktur und interessanten makroskopischen physikalischen anisotropen Eigenschaften erzeugt werden.

Wie bereits ausgeführt, kann die Permeabilität der Polyelektrolythülle durch Modifikationen, z.B. Aufbringen von Lipidschichten, gesteuert werden. Dies kann für pharmazeutischen Anwendungen genutzt werden, indem nach der Verkapselung polarer niedermolekularer Substanzen Lipide auf die Hülle aufgetragen werden, um auf diese Weise die Permeabilität der Hülle für die verkapselten Substanzen zu vermindern. Der verkapselte Stoff kann nur noch langsam durch die Lipidschicht mit einer über lange Zeit konstanten Rate austreten, was für pharmakologische Applikationen oftmals wünschenswert ist.

Durch die Verkapselung und anschließende Auflösung von Templaten können formgetreue dreidimensionale Abdrücke von Templatpartikeln hergestellt werden. Bei Blockvernetzung der Polyelektrolythüllen erhält man mesoporöse Materialien mit monodisperser formgetreuer Porenverteilung. Diese Materialien besitzen eine hohe innere Oberfläche verbunden mit hoher Festigkeit und machen sie zu hervorragenden Filtersubstanzen für industrielle Zwecke. Durch Auswahl der Template (Form und Größe) können mesoporöse Materialien mit vorgegebenen Poren hergestellt werden.

Natürlich kann durch Variation der für die Herstellung der Polyelektrolythülle verwendeten Materialien auch die Oberflächenchemie in weiten Bereichen variiert werden.

Schließlich können die Polyelektrolythüllen auch zur Erzeugung von pH-Gradienten zwischen dem inneren der Hülle und dem die Hülle umgebenden Volumen verwendet werden. Dieser pH-Gradient kann wiederum zu effizienten Beladungen der Hüllen mit Wirkstoffen ausgenutzt werden.

Weiterhin betrachtet wird das Aufbringen mehrerer aufeinanderfolgender Schichten auf einen Träger durch ein Filtrationsverfahren. Durch diese Methode können mit Polyelektrolytmoleküle beschichtete Kapseln effizient, auf einfache Weise und in großem Maßstab hergestellt werden. Überraschenderweise können selbst empfindliche Templatpartikel wie biologische Zellen durch ein Filtrationsverfahren beschichtet werden.

Die Erfindung betrifft somit ein Verfahren zum Aufbringen mehrerer Schichten von Beschichtungssubstanzen auf Templatpartikel umfassend die Schritte:
(a) Inkontraktbringen des Templatpartikels mit einer ersten Beschichtungssubstanz in einem fluiden, vorzugsweise wässrigen Reaktionsmedium in einem Reaktionsraum, der auf mindestens einer Seite durch eine Filtrationsmembran begrenzt ist, unter Bedingungen, bei denen eine Schicht der ersten Beschichtungssubstanz auf dem Templatpartikel gebildet wird,
(b) Ableiten von mindestens einem Teil des Reaktionsmediums mit gegebenenfalls darin enthaltener überschüssiger erster Beschichtungssubstanz durch die Filtrationsmembran in einen Filtratraum, wobei vorzugsweise die überschüssige erste Beschichtungssubstanz im wesentlichen vollständig abgeleitet wird,
(c) Inkontaktbringen des Templatpartikels mit einer zweiten Beschichtungssubstanz in einem fluiden Reaktionsmedium in einem Reaktionsraum der auf mindestens einer Seite durch eine Filtrationsmembran begrenzt ist, unter Bedingungen, bei denen eine Schicht der zweiten Beschichtungssubstanz auf dem Templatpartikel gebildet wird,
(d) Ableiten von mindestens einem Teil des Reaktionsmediums mit gegebenenfalls darin enthaltener überschüssiger zweiter Beschichtungssubstanz durch die Filtrationsmembran in einen Filtratraum, wobei vorzugsweise die überschüssige zweite Beschichtungssubstanz vorzugsweise im wesentlichen vollständig abgeleitet wird, und
(e) gegebenenfalls mehrmaliges Wiederholen der Schritte (a) und (b) oder/und (c) und (d).

Als erste und zweite Beschichtungssubstanzen werden vorzugsweise jeweils entgegengesetzt geladene Polyelektrolytspezies oder Gemische von Polyelektrolyspezies verwendet. Weiterhin können als Beschichtungssubstanzen auch Nanopartikel, amphiphile Polyelektrolyte, Lipide oder/und Tenside verwendet werden.

Ein weiterer Gegenstand der Erfindung ist ein Verfahren zur Modifizierung der Permeabilität von Polyelektrolythüllen, dadurch gekennzeichnet, dass auf der Polyelektrolythülle Lipidschichten abgeschieden werden.

Die Templatpartikel werden vorzugsweise aus Partikeln mit einem Durchmesser von bis zu 10 µm ausgewählt.

Vorzugsweise werden auflösbare Partikel wie zuvor genannt, z.B. teilvernetzte Melaminformaldehydpartikel, biologische Partikel oder Aggregate biologischer oder/und amphiphiler Materialien, insbesondere biologische Aggregate wie Zellen, Zellaggregate, Viruspartikel etc. verwendet.

Um eine vollständige Entfernung überflüssiger Beschichtungssubstanz nach einem Beschichtungsschritt zu ermöglichen, wird während oder/und nach Schritt (b) oder/und (d) ein Waschmedium, z.B. Wasser oder eine wässrige Pufferlösung in den Reaktionsraum eingebracht. Insbesondere bei empfindlichen Templatpartikeln wie biologischen Aggregaten erfolgt die Zugabe des Waschmediums derart, daß das im Reaktionsraum befindliche Volumen des Mediums gemäß einem vorgegebenen Programm gesteuert wird, z.B. in Schritt (b) oder/und Schritt (d) im wesentlichen konstant bleibt.

Die Schritte (a) und (c) können jeweils im gleichen Reaktionsraum, aber auch in unterschiedlichen Reaktionsräumen durchgeführt werden. Die Filtrationsmembranen werden zweckmäßigerweise so gewählt, daß sie einerseits in der Lage sind, partikelförmige Templatmaterialien zurückzuhalten, aber andererseits eine rasche Entfernung des verbrauchten Reaktionsmediums ermöglichen. Beispiele für geeignete Filtermaterialien sind Polyamid, Cellulosenitrat und Celluloseacetat. Um bei empfindlichen Templatpartikeln eine Aggregatbildung oder/und ein Verstopfen des Filters zu vermeiden, wird das Verfahren unter Bedingungen durchgeführt, welche die Adhäsion von Templatpartikeln unterdrücken. So können gegebenenfalls für jeden Filtrationsschritt Membranen verwendet werden, die eine gleichsinnige Ladung wie die im jeweiligen Schritt verwendete Polyelektrolytspezies haben.

Die Filtration kann durch Anlegen eines Überdrucks im Reaktionsraum oder/und eines Vakuums im Filtratraum beschleunigt werden. Bei empfindlichen Templatpartikeln, insbesondere biologischen Aggregaten, wird die Filtration im wesentlichen ohne Druckdifferenz (Druckdifferenz ≤ ± 0,5 bar) zwischen Reaktionsraum und Filtratraum durchgeführt. Weiterhin ist ein Rühren des Reaktionsraumes zumindest während der Schritte (a) oder/und (c), insbesondere ein kontinuierliches Rühren während des gesamten Prozesses in vielen Fällen vorteilhaft.

Das betrachtete Membranfiltrationsverfahren ist kontinuierlich durchführbar, erlaubt die Produktion größerer Mengen an beschichteten Partikeln in kürzester Zeit, ist visuell kontrollierbar und verhindert weitestgehend die Aggregatbildung von Teilchen. Das Verfahren kann im großtechnischen Maßstab durchgeführt werden und kann aufgrund seiner Flexibilität an verschiedene Anforderungen der speziellen Partikel und Beschichtungssysteme adaptiert werden. Bei Verwendung löslicher Templatpartikel kann der Abbau der Kerne kontinuierlich im Anschluß an die Beschichtung erfolgen.

Die Erfindung wird durch die beigefügten Figuren und Beispiele weiter erläutert.
- Fig.1: stellt eine schematische Veranschaulichung einer bevorzugten Ausführungsform des erfindungsgemäßen Verfahrens dar.
- Fig.2: zeigt die Schichtdicke als Funktion der Zahl der Schichten bei der Absorption von Poly(allylaminhydrochlorid) (PAH) und Poly(styrolsulfonat, Natriumsalz) (PSS) auf negativ geladene Polystyrol (PS)-Latexpartikel.
- Fig.3: zeigt ein SEM-Abbild (Rasterelektronenmikroskopie) einer Polyelektrolythülle mit neun Schichten (PSS/PAH)₄/PSS nach Auflösung des Kerns. Die äußere Schicht ist PSS.
- Fig.4: zeigt ein TEM-Abbild (Transmissionselektronenmikroskopie) einer Polyelektrolythülle mit neun Schichten (PSS/PAH)₄/PSS.
- Fig.5: zeigt atomkraftmikroskopische Abbildungen von PSS/PAH-Polyelektrolythüllen. Die Zahl der Polyelektrolythüllen beträgt in Fig.5(A) 3 PSS/PAH/PSS und in Fig.5(B) 9 (PSS/PAH)₄/PSS].
- Fig.6: zeigt ein AFM-Abbild von PS-Latexteilchen mit 1,28 µm Durchmesser und einer Polyelektrolythülle mit sechs Schichten (PAH/PSS)₃. Die äußere Schicht ist PSS.
- Fig.7: zeigt normalisierte Lichtstreuungs-Intensitätsverteilungen von PAH/PSS-beschichteten PS-Latexpartikeln. Partikel mit 11 und 21 Schichten werden mit den unbeschichteten Partikeln verglichen.
- Fig.8: zeigt ein TEM-Abbild eines PS-Latexpartikels beschichtet mit vier Schichten PAH/PSS und anschließend drei Schichtpaaren bestehend jeweils aus einer Schicht Magnetit und einer Schicht PSS. Die Skala entspricht 200 nm.
- Fig. 9: zeigt ein AFM-Abbild einer Polyelektrolythülle erzeugt durch Beschichtung eines 3,7 µm MF-Latexpartikels mit 10 Schichten PSS/PAH und anschließende Auflösung des Templatpartikels.
- Fig.10: zeigt ein AFM-Abbild von Polyelektrolythüllen erhalten durch Beschichtung von Glutardialdehyd-fixierten humanen Erythrozyten mit zehn Schichten PSS/PAH und anschließende Auflösung des Templatpartikels. Die Skalenwerte der Achsen sind in µm und die Werte der Höhenskala in nm angegeben.
- Fig.11: zeigt ein AFM-Abbild von Polyelektrolythüllen erhalten durch Beschichtung von 3,7 µm MF-Latexpartikeln mit zehn Schichten Chitosan/Chitosansulfat und anschließende Auflösung des Templatpartikels.
- Fig.12: zeigt ein CLSM-Abbildung von Chitosan/Chitosansulfatmikrokapseln bestehend aus elf Schichten, wobei als äußerste Schicht FITC-markiertes PAH verwendet wurde.
- Fig.13: zeigt das Zetapotential von beschichteten und mit Chitosan/-Chitosansulfat beschichteten 3,7 µm MF-Latexpartikeln.
- Fig.14: zeigt die Fluoreszenzintensität einer 6-CF als Fluoreszenzmarker enthaltenden Suspension von Polyelektrolythüllen gegenüber dem pH-Wert des umgebenden Mediums die durch HCl oder H-PSS mit dem jeweils angegebenen Molekulargewicht titriert wurde.
- Fig.15: zeigt die Beziehung des pH-Werts im Inneren der Kapseln zum pH-Wert des umgebenden Mediums, titriert mit H-PSS (MW 4200) in Abwesenheit von Salz (ausgefüllte Kreise) und in Gegenwart von 1 mM NaCl (leere Kreise). Die gestrichelte Linie zeigt die durch Titration der Kapseldispersion mit HCl erhaltene Kontrolle.

### Beispiele

### Beispiel 1 Herstellung von teilvernetzten Melaminformaldehyd-Templat-partikeln

Monodisperse Melaminformaldehydpolymerpartikel können durch eine Polykondensationsreaktion aus Melaminformaldehydpräkondensaten im Größenbereich bis zu 15 µm hergestellt werden (vgl. DD 224 602). Die Größe der Partikel kann durch die Monomerkondensation, den pH-Wert, die Reaktionstemperatur und den Tensidzusatz beeinflußt werden. Bei den im Stand der Technik beschriebenen Verfahren werden hochvernetzte Partikel erhalten, die in den meisten organischen Lösungsmitteln, wie etwa Xylol, Toluol und Alkohol sowie in Säuren und Basen unlöslich sind. Zur Herstellung von auflösbaren, teilvernetzten Melaminformaldehyd-Templat-Partikeln wird das im Stand der Technik beschriebene Verfahren modifiziert, indem der Polykondensationsprozeß auf einer bestimmten Initialstufe der Reaktion unterbrochen wird. Dadurch werden in wäßrigen Medien lösliche Kerne erhalten. Der Abbruch der Reaktion kann durch rasche Temperaturabsenkung, durch Veränderung des pH-Werts in den alkalischen Bereich und durch Wahl geeigneter Präkondensate, insbesondere Tetramethylolmelamin, erreicht werden.

Die so erhaltenen Kerne können durch Säurezugabe und/oder durch bestimmte chemische Reaktionen, insbesondere Sulfonierung in wäßrigen Medien, aufgelöst werden. Als Sulfonierungsagentien können insbesondere Alkalisulfite, Alkalihydrogensulfite und andere wasserlösliche Salze der schwefligen Säure eingesetzt werden. Die Auflösbarkeit der Kerne kann durch den Zeitpunkt der Unterbrechung des Polykondensationsprozesses beeinflußt werden. Die Unterbrechung wird 1 min bis 3 h nach Start der Reaktion in Abhängigkeit der Reaktionsbedingungen und in Abhängigkeit der gewünschten Auflösbarkeit der Kerne durchgeführt. Die Auflösungsgeschwindigkeit kann weiterhin durch die Wahl von pH-Wert, Temperatur und Sulfonierungsreagens gesteuert werden. Somit ist es möglich, Kerne mit einer Auflösegeschwindigkeit von 0,1 s bis 10 h, wiederum in Abhängigkeit der Auflösungsbedingungen, zu erhalten. Diese auflösbaren Melaminformaldehydpartikel werden hierin als teilvernetzte Melaminformaldehydpartikel bezeichnet.

### Beispiel 2 Herstellung von leeren Polyelektrolythüllen unter Verwendung von Melaninformaldehydpartikeln als Templat

### 2.1

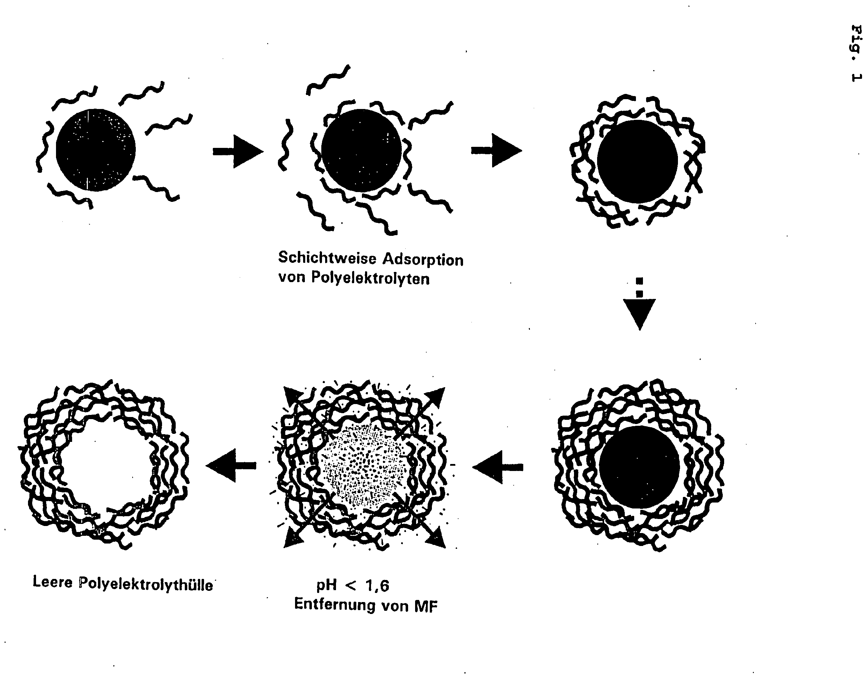

Auf wie in Beispiel 1 beschrieben hergestellte monodisperse, kolloidale, teilvernetzte Melaminformaldehydpartikel (MF) mit einem Durchmesser von 2,0 bzw. 3,3 µm werden schrittweise Polyelektrolyte aus verdünnten wäßrigen Lösungen aufgebracht (vgl.Fig.1). Die Polyelektrolytschichten werden durch alternierende Adsorption von entgegengesetzt geladenen Polyionen, beginnend mit der Adsorption eines negativ geladenen Polyanions (z.B. Polystyrolsulfonat, Natriumsalz; PSS) auf die positiv geladenen MF-Partikel aufgebracht. Typische Adsorptionsbedingungen waren 20 mM Polyelektrolyt (Konzentrationsangabe bezogen auf Monomer) 0,5 M NaCl bei Partikelkonzentrationen von 0,5 Gew.-%. Die Adsorptionsdauer betrug 20 min. Die MF-Partikel hatten eine Dichte von 1,5 g/cm³.

Nach Beendigung dieses Adsorptionszyklus wurde überschüssiger Elektrolyt durch wiederholte Zentrifugations/Waschzyklen entfernt. Dazu wurden die beschichteten Kerne bei einer Zentrifugationsgeschwindigkeit von 2000 U/min (unter Verwendung eines Eppendorf-Rotors) abgesetzt. Dann wurden drei Waschschritte mit entionisiertem Wasser vor der Zugabe des nächsten Polyelektrolyten durchgeführt, um die vollständige Entfernung von nichtadsorbiertem Polyelektrolyten sicherzustellen. Durch Wiederholung dieser Vorgehensweise kann die gewünschte Zahl von Polyelektrolytschichten aufgebracht werden.

Anschließend wurde der pH-Wert auf < 1,6 erniedrigt, wodurch die MF-Kerne innerhalb weniger Sekunden aufgelöst werden. Die Fragmente dringen durch die Poren der Hülle nach außen und können entfernt werden, so daß eine leere Polyelektrolythülle erhalten wird. Bei pH-Werten oberhalb 1,8 wird keine nachweisbare Auflösung der Kerne beobachtet.

### 2.2

100 µl einer 3%-igen Dispersion von teilvernetzten MelaminformaldehydPartikeln mit einer Partikelgröße von 3 µm werden mit 400 µl einer Lösung von 20 mono mM PSS in 0,5 M NaCl versetzt. Nach einer Einwirkzeit von 5 min unter leichtem Schütteln wird 1 ml reines Wasser zugesetzt. Nach Zentrifugation bei 2000 U/min wird der Überstand decantiert, das Sediment mit reinem Wasser aufgefüllt und die Zentrifugation wiederholt. Abermaliges Decantieren und ein weiterer Zentrifugationszyklus ergeben gereinigte, mit einer PSS-Schicht bedeckte MF-Partikel. In analoger Weise wird anschließend eine Poly(allylaminhydrochlorid)-Schicht (PAH) aufgebracht. Diese Zyklen werden abwechselnd in Abhängigkeit von der gewünschten Zahl der Schichten wiederholt. Nach dem Zentrifugationszyklus am Ende des Aufbaus der letzten Schicht werden 1 ml einer 0,1 N Salzsäure zugegeben. Nach etwa 5 min Schütteln erhält man eine klare Lösung, da die durch die Partikel verursachte Trübung der Lösung verschwunden ist. Anschließend zentrifugiert man für etwa 10 min bei 10 000 U/min. Bei dieser Zentrifugation scheidet sich ein feiner, leicht milchig wirkender Bodensatz ab, der die gebildeten Polyelektrolythüllen enthält. Bereits ein leichtes Schütteln nach Zusatz von Wasser ist ausreichend um die Hüllen zu resuspendieren. Nach zwei weiteren Zentrifugationsschritten erhält man eine gereinigte, 3%-ige Dispersion von sphärischen, monodispersen Polyelektrolythüllen in Wasser. Eine Probe dieser Hüllen kann durch Rasterelektronenmikroskopie, Transmissionselektronenmikroskopie und/oder Atomkraftmikroskopie untersucht werden.

### 2.3

1,59 mg Na-Polystyrolsulfonat (PSS) werden einer Dispersion von teilvernetzten Melaminformaldehydpartikeln in 0,5 M NaCl zugesetzt. Die MF-Dispersion enthält insgesamt 2,2 x 10⁸ Partikel. Nach zwanzigminütigem leichtem Schütteln werden 0,81 mg Polyallylaminhydrochlorid zugesetzt. Nach wiederum 20 min werden unter leichtem Schütteln 1,59 mg PSS zugegeben. Diese Vorgehensweise wird 5x mit jeweils einer PAH- und einer PSS-Zugabe wiederholt. Man erhält dadurch Melaminformaldehydpartikel, die mit 13 alternierenden Schichten bedeckt sind. Durch Zugabe von 10 ml 1 N-Salzsäure wird der pH-Wert erniedrigt, so daß sich die MF-Kerne auflösen. Durch die Zentrifugation bei 15 000 g für 15 min werden die Polyelektrolythüllen vom Überstand abgetrennt.

### Beispiel 3 Charakterisierung der Polyelektrolythüllen

### 3.1 Abhängigkeit der Schichtdicke von der Anzahl der Schichten

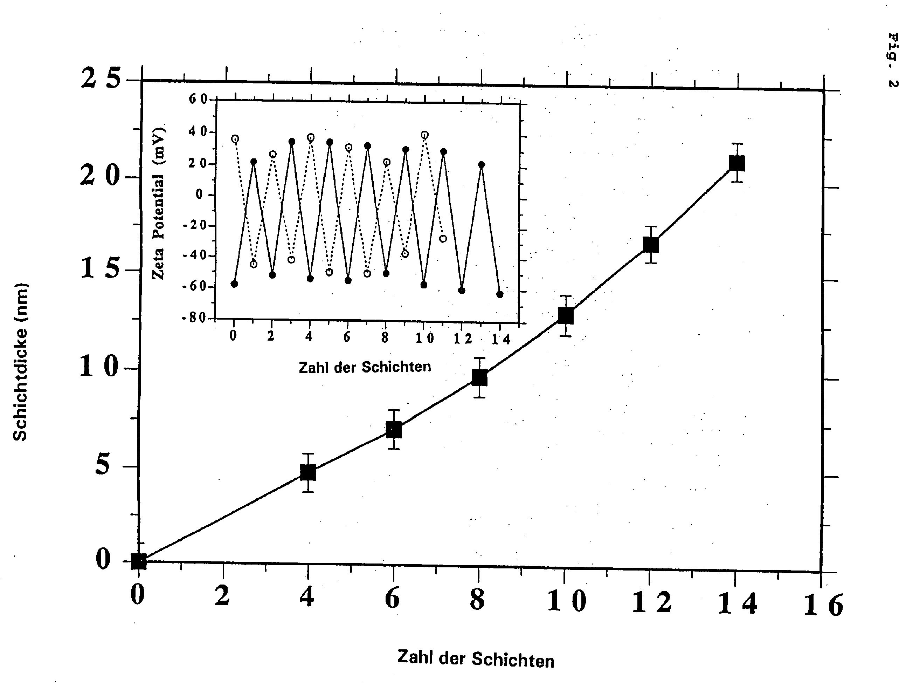

Auf negativ geladene Polystyrol (PS)-Latexpartikel wurden abwechselnd Schichten aus Poly(allylaminhydrochlorid) (PAH) und Poly(styrolsulfonat, Natriumsalz) (PSS) adorbiert. Die Schichtdicke wurde durch Einzelteilchen-Lichtstreuung gemessen. Der Intensitätsanstieg des gestreuten Lichts ist ein Maß für die adsorbierte Menge und wurde in die Schichtdicke unter Verwendung des Refraktionsindex der Polyeletrolytschichten konvertiert. Das Insert in Fig.2 gibt das aus elektrophoretischen Mobilitätsmessungen (Malvern Zetasizer 4) abgeleitete Zetapotential für die Adsorption von PAH und PSS auf Polystyrolpartikel (ausgefüllte Kreise) und für die Adsorption von PSS und PAH auf positiv geladene MF-Partikel (offene Kreise) an.

Das Zetapotential ist ein Maß für die effektive Ladungsdichte auf der Partikeloberfläche. Wie aus Fig.2 ersichtlich ist, tritt eine Umkehrung des Oberflächenpotentials mit der Adsorption jeder Polyelektrolytschicht auf die Polystyrol- bzw. MF-Partikel auf. Eine Umkehrung des Oberflächenpotentials begünstigt die anschließende Adsorption des entgegengesetzt geladenen Polyions.

Flugzeitmassenspektrometrische Untersuchungen haben gezeigt, daß bei der Auflösung der teilvernetzten MF-Templatpartikel bei einem pH-Wert < 1,6 MF-Oligomere gebildet werden, die hauptsächlich aus 5-10 Einheiten Tetramethylolmelamin bestehen. Diese MF-Oligomere haben eine charakteristische Querschnittsausdehnung von etwa 1 nm, wie durch molekulare Dynamiksimulationen bestimmt (unter Verwendung des Programms DISCOVERY). Diese Oligomere werden aus dem Kern ausgestoßen und permeiren durch Polyelektrolytschichten, die die Hülle bilden, und können schließlich von den leeren Schalen durch Zentrifugation abgetrennt werden. Dies bestätigt, daß die Hüllen für Moleküle mit einer Größe im Bereich von wenigen nm, insbesondere ≤ 10 nm, bevorzugt ≤ 5 nm, leicht permeabel sind.

### 3.2 Rasterelektronenmikroskopische Untersuchungen der Polyelektrolythüllen

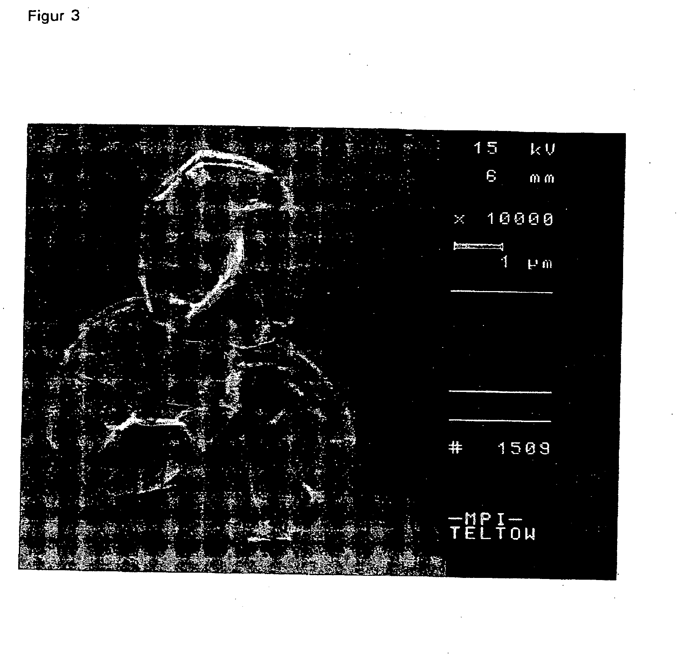

Die Polyelektrolythüllen wurden mittels Rasterelektronenmikroskopie (SEM) untersucht. Zunächst wurde ein MF-Kern mit einem Durchmesser von 3,3 µm mit 9 Polyelektrolytschalen (PSS/PAH)₄/PSS beschichtet. Die äußerste Schicht ist PSS. Nach Auflösung des MF-Kerns wurden die erhaltenen Kapseln mit SEM untersucht. Wie aus Fig.3 ersichtlich, liegen die Durchmesser im Bereich von 4,0 ± 0,5 µm. Die Schalen werden durch eine starke elektrostatische Anziehung an die positiv geladene, Poly(ethylenimin)-beschichtete Glasoberfläche immobilisiert. Weiterhin tritt während der Untersuchung ein gewisses Ausmaß an Austrocknen der Kapseln ein. Dies führt dazu, daß die Hülle faltig wird. Wie jedoch aus Fig.3 ersichtlich ist, sind keine Löcher oder Rißspuren in den Hüllen zu finden.

Die SEM-Messungen wurden unter Verwendung eines Zeiß-DSM40-Instruments durchgeführt, welches bei einer Beschleunigungsspannung von 15 KeV betrieben wurde. Die Proben wurden durch Aufbringen eines Tropfens einer die Hüllen enthaltenden Lösung auf Poly(ethylenimin)-beschichtetes Glas hergestellt. Nachdem sich die Hüllen auf den Glasträgern abgesetzt hatten, wurden sie gründlich mit Wasser gespült und vorsichtig unter einem Stickstoffstrom getrocknet.

### 3.3 Transmissionselektronenmikroskopie (TEM)

Auf MF-Templatpartikel mit einem Durchmesser von 2 µm wurden neun Schichten Polyelektrolyt (PSS/PAH)₄/PSS aufgebracht. Anschließend wurden die Templatpartikel aufgelöst. Die Proben wurden mit Glutardialdehyd, OsO₄ und K₂Cr₂O₇ fixiert, in Ethanol/Aceton dehydriert, in ein Epon 812/Araldit M-Harz eingebettet und in einem Ofen für zwei Tage polymerisiert. Dünnschnitte (80 bis 100 nm) wurden unter Verwendung eines Reichert-Ultratom angefertigt und mit Uranylacetat und Bleicitrat eingefärbt.

Die Messungen wurden auf einem JEOL 100 B Elektronenmikroskop durchgeführt.

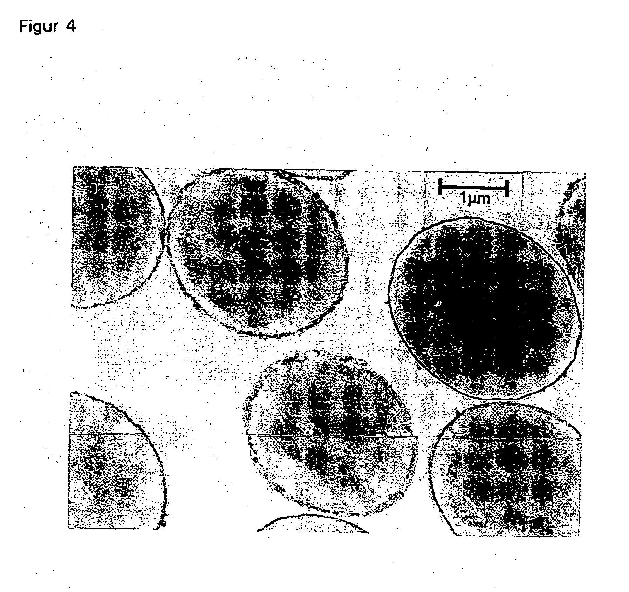

Wie aus Fig.4 ersichtlich, kann die angefärbte Polyelektrolytschicht, die das weniger angefärbte Zellinnnere umgibt, deutlich identifiziert werden. Die homogene Form der Hüllen zeigt, daß die hergestellten Kapseln sowohl den Durchmesser als auch die sphärische Form der Templatpartikel beibehalten, unter der Voraussetzung, daß die innere wäßrige Lösung nicht entfernt wird. Weiterhin ist zu erkennen, daß die Dicke der aus neun Schichten bestehenden Polyelektrolythülle in der Größenordnung von 20 nm ist. Dieser Wert stimmt mit den in Fig.2 gezeigten Daten für Polyelektrolyt-beschichtete Polystyrolpartikel überein. Daraus kann man schließen, daß die Art der Templatpartikel die Dicke der Polyelektrolytschichten nicht wesentlich beeinflußt. Aus dem TEM-Abbild ist auch ersichtlich, daß die Polyelektrolythüllen weder Risse noch Löcher aufweisen.

### 3.4 Atomkraftmikroskopische (AFM) Untersuchungen

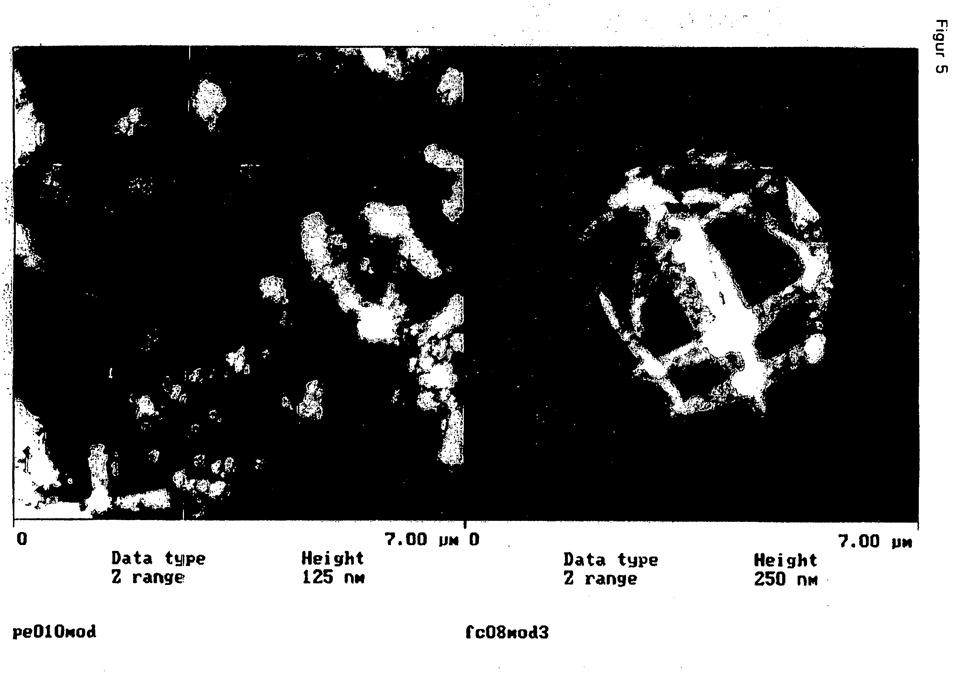

PSS/PAH-Polyelektrolythüllen wurden unter Verwendung von MF-Templatpartikeln mit einem Durchmesser von 3,3 µm wie oben beschrieben hergestellt. Die Zahl der Polyelektrolytschichten betrug 3 PSS/PAH/PSS (Fig.5(A)) bzw. 9 (PSS/PAH)₄/PSS (Fig.5(B)). Diese Kapseln wurden mit AFM im Tapping Mode (TM) untersucht. Fig.5 zeigt, daß die dreidimensionalen Polyelektrolythüllen durchgehende Folien sind, die Falten aufweisen, die von der Verdampfung des wäßrigen Inneren resultieren. Wie zu sehen ist, steigt die Höhe der Kapseln mit ansteigender Schichtzahl an. Die maximale Höhe der getrockneten Hüllen in Abbildung A liegt in der Größenordnung von 50 nm und in Fig.5(B) in der Größenordnung von 100 nm.

### Beispiel 4 Herstellung von trägerfixierten Polyelektrolythüllen

Ein sorgfältig gereinigter Glasträger wird 5 min in eine wäßrige Lösung von 0,5 mg/ml Polyethylenimin eingetaucht. Danach bläst man den Glasträger in einem Stickstoffstrom trocken. 100 µl einer 3%-igen Dispersion aus teilvernetzten Melaminformaldehydpartikeln mit einer Partikelgröße von 1 µm Durchmesser werden mit 400 µl einer 20 mono-mM Na-Poly(styrolsulfonat)-lösung NaCl versetzt. Nach 5 min unter leichtem Schütteln wird 1 ml reines Wasser zugesetzt. Nach Zentrifugation bei 2000 U/min wird der Überstand decantiert, das Sediment mit reinem Wasser aufgefüllt und die Zentrifugation wiederholt. Nach abermaligem Decantieren und einem weiteren Zentrifugationszyklus erhält man mit einer PSS-Schicht bedeckte MF-Partikel. Anschließend gibt man 400 µl einer 20 mono-mM-Polydiallyldimethylammoniumchloridlösung in 0,5 M NaCl zu den Partikeln und inkubiert für 20 min Diesen Vorgang wiederholt man ein zweites Mal. Anschließend werden die Partikel wiederum wie oben beschrieben mit PSS beschichtet und dreimal zentrifugiert. Das Sediment wird in 0,5 ml reinem Wasser redispergiert und auf den Glasträger aufgebracht. Nach 5 min taucht man den Glasträger für 5 min in eine 0,1 N Salzsäurelösung. Danach taucht man das Glasplättchen ohne Zwischentrocknung für jeweils 5 min dreimal in reines Wasser. Danach wird das Glasplättchen in einem leichten Stickstoffstrom getrocknet. Als Resultat erhält man dichtgepackte, auf einem Polyethylenimin-beschichteten Glasträger fixierte Polyelektrolythüllen, die aus 5 Schichten bestehen.

### Beispiel 5 Einschluß von Wirkstoffen in Polyelektrolythüllen

100 µl einer 2%-igen Dispersion aus teilvernetzten Melaminformaldehydpartikeln mit einer Partikelgröße von 0,9 µm Durchmesser werder mit 400 µl einer 0,5 M NaCl-Lösung, pH 6, versetzt, die 0,5 mg/ml Polymilchsäure enthält. Nach 5 min unter leichtem Schütteln wird 1 ml Wasser zugesetzt. Nach Zentrifugation bei 2000 U/min (5 cm Rotorradius) wird der Überstand decantiert, Wasser nachgefüllt und die Zentrifugation wiederholt. Nach abermaligem Decantieren und einem weiteren Zentrifugationszyklus werden mit einer Polymilchsäureschicht bedeckte Melaminpartikel erhalten. Diese werden mit 0,4 ml einer 1 mg/ml Lysozymlösung bei pH 6,0 versetzt und für 20 min bei leichtem Schütteln inkubiert. Anschließend wird dreimal in Wasser gewaschen. Bei pH 6 wird eine weitere Polymilchsäureschicht wie oben beschrieben aufgebracht. Anschließend wird eine Poly(allylaminhydrochlorid)schicht (PAH) aufgebracht, danach weitere Schichten in der Folge PSS/PAH/PSS.

Anschließend überführt man die Partikel in eine 0,1 N Salzsäurelösung. Nach wenigen Sekunden bilden sich unter Auflösung der Kerne und der zwei Polymilchsäureschichten mit Lysozym gefüllte Polyelektrolythüllen aus. Diese werden zweimal bei 15 000 g in reinem Wasser zentrifugiert. Der Überstand wird jeweils verworfen. Man erhält als Sediment konzentrierte, mit Lysozym, einem Protein, gefüllte Kapseln, die eine Polyelektrolythülle aus 4 Schichten aufweisen. Andere biologische Makromoleküle können auf ähnliche Weise verkapselt werden.

### Beispiel 6 Herstellung von leeren Polyelektrolythüllen unter Verwendung biologischer Partikel als Templat

Rinder- oder Humanerythrozyten werden mit Glutadialdehyd in einer Konzentration von 2% fixiert. Nach 60 min Einwirkzeit bei 20°C wird die Lösung abzentrifugiert und die Erythrozyten werden viermal in bidestilliertem Wasser gewaschen. Anschließend werden die fixierten Erythrozyten mit ungepufferter 154 mM NaCl-Lösung aufgefüllt.

Zur Beschichtung werden 4 ml Lösung mit einer Konzentration von 0,5 g/dl PAH und 0,5 M NaCl bei einer Erythrozytenkonzentration von ca. 2,5% (v/v) angesetzt. Nach 10 min Einwirkzeit bei 20°C werden die Erythrozyten abzentrifugiert und zweimal in einer 154 mM NaCl Lösung gewaschen. Anschließend werden 4 ml Lösung mit einer Konzentration von 0,5 g/dl PSS und 0,5 m NaCl und einer Erythrozytenkonzentration von ca. 2,5% (v/v) angesetzt. Nach 10 min Einwirkzeit bei 20°C werden die Erythrozyten abzentrifugiert und zweimal in einer 154 mM NaCl-Lösung gewaschen. Das Aufbringen von PAH und PSS Schichten kann beliebig oft wiederholt werden.

Die Auflösung des Templats kann in einer 1,2%-igen NaOCl Lösung erfolgen. Ebenso geeignet sind handelsübliche Deproteinisierungsmittel (Medical Instruments) oder Abflußreiniger (z.B. Chlorix). Die Einwirkzeit beträgt ca. 20 min bei 20°C und läßt sich optisch über die verschwindende Trübung der Lösung kontrollieren. Die verbleibenden Polymerhüllen werden anschließend in NaCl-Lösung gewaschen.

Auf analoge Weise können auch E.coli oder Hefezellen beschichtet werden. Auch nichtfixierte Zellen können beschichtet werden.

### Beispiel 7 Abscheidung von Lipidschichten auf Polyelektrolythüllen

Zur Abscheidung von Lipidschichten auf Polyelektrolythüllen wurden zwei unterschiedliche Verfahren verwendet.

### 7.1

200 µl einer Suspension von Polyelektrolythüllen werden durch wiederholtes Waschen in Methanol resuspendiert. Nach dem dritten Waschen werden anstelle von reinem Methanol 500 µl einer Lipidlösung von z.B. 1 mg/ml Dipalmitoylphosphatidinsäure (DPPA) oder Dipalmitoylphosphatidylcholin (DPPC) in Methanol dem Sediment zugesetzt. Die Hüllen werden in dieser Methanol-Lipidlösung resuspendiert und die Suspension bei einer Temperatur von 90°C in einem Wasserbad gehalten. Der verdampfende Methanol wird durch tropfenweise Zugabe von Wasser in Portionen von jeweils 20 µl ersetzt. Der Austausch von 700 µl Methanol gegen Wasser dauert etwa 30 min.

Nach Beendigung der Verdampfung wird die Hüllensuspension dreimal mit Wasser gewaschen und wiederholt zentrifugiert. Durch 20 min Zentrifugation bei 25 000 Upm können die Lipid-beschichteten Hüllen sedimentiert werden.

### 7.2

Dispersionen von DPPA oder 90% DPPC und 10% DPPA mit einer Konzentration von 1 mg Lipid/ml in Wasser werden durch Ultraschallbehandlung hergestellt. 500 µl der resultierenden Dispersion von Lipidvesikeln werden auf 200 µl einer konzentrierten Hüllensuspension gegeben. Nach 30 min werden die Proben 20 min bei 25 000 Upm zentrifugiert. Der Überstand wird entfernt und durch Wasser ersetzt. Diese Prozedur wird dreimal wiederholt. Dabei wird eine konzentrierte Suspension von Lipid-beschichteten Hüllen erhalten.

### Beispiel 8 Einschluß von organischen Lösungsmitteln in Polyelektrolythüllen

Eine wässrige Suspension von Polyelektrolythüllen wird 5 min bei 3000 Upm zentrifugiert. Nach Entfernen des Überstands wird Methanol zugegeben. Die Hüllen werden resuspendiert und 10 min lang bei 4000 Upm zentrifugiert. Erneut wird der Überstand entfernt, Methanol zugegeben und die Probe unter denselben Bedingungen wie zuvor zentrifugiert. Diese Prozedur wird dreimal wiederholt. Nach der letzten Zentrifugation mit Methanol wird der Überstand durch Hexanol ersetzt. Die Hüllen werden resuspendiert und 10 min bei 5000 Upm zentrifugiert. Diese Prozedur wird wiederum dreimal wiederholt.

Zum Einschluß von Octanol, Octan oder Decan in die Hüllen wird eine ähnliche Prozedur verwendet, wobei als Ausgangsmaterial die in einer Hexanollösung vorliegenden Hüllen verwendet werden. Die Zentrifugationsgeschwindigkeit wird für Octanol und Octan auf 7000 Upm (10 min) und für Decan auf 7500 Upm (10 min) erhöht.

Schließlich wird das resultierende Sediment in Wasser resuspendiert. Die Hüllen verbleiben in der wässrigen Phase, während die noch vorhandenen Spuren des Lösungsmittels im Sediment zwischen den Hüllen eine zweite organische Phase bilden. Durch Verwendung von Fluoreszenzmarkern für die organische und die wässrige Phase kann mittels konfokaler Mikroskopie gezeigt werden, daß die Hüllen mit organischem Lösungsmittel gefüllt sind.

Die beschriebene Prozedur ermöglicht die Herstellung einer hochstabilen Emulsion von nichtpolaren Flüssigkeiten in Wasser. Als Folge der Monodispersität der ursprünglichen Hüllen ist die erzeugte Emulsion ebenso monodispers. Ein weiterer Vorteil ist, daß selbst die Form der einzelnen Tröpfchen - abhängig vom verwendeten Templat - reguliert werden kann. Dies ermöglicht die Herstellung von Emulsionen mit Oberfläche:Volumen-Verhältnissen, die von denjenigen einer Kugel verschieden sind.

### Beispiel 9 Präzipitation und Kristallisation in Polyelektrolythüllen

Die leeren Polyelektrolythüllen können auch zur kontrollierten Präzipitation oder Kristallisation organischer oder anorganischer Materialien verwendet werden. Hierzu werden Polyelektrolythüllen in einer 30 mM 6-Carbofluorescein (6-CF) Lösung bei pH 7 inkubiert. Anschließend wird der pH-Wert der Lösung rasch auf einen Wert von 3,5 verändert, bei dem 6-CF weitgehend unlöslich wird. Nach Inkubation über 1 bis 12 h wird eine Mischung von vollständig mit 6-CF gefüllten und leeren Hüllen erhalten. In weiteren Experimenten konnte Rhodamin B in Polyelektrolythüllen durch Erhöhung des pH-Werts präzipitiert werden.

Die Präzipitation von Wirkstoffen kann auch durch andere Maßnahmen, z.B. Lösungsmittelaustausch, Salzfällung, etc. ausgelöst werden. Diese Ergebnisse zeigen, daß die Polyelektrolythüllen als Template für Kristallisations- oder Präzipitationsprozesse dienen können, wodurch eine Kontrolle der Größe und Form von durch die Reaktion entstandenen kolloidalen Teilchen ermöglicht wird.

### Beispiel 10 Polymerisation in Polyelektrolythüllen

Eine 3% Lösung von Diallyldimethyldiammoniumchlorid (DADMAC) wird in einer 2%-igen Suspension von Polyelektrolythüllen mit dem Polymerisationsinitiator Natriumperoxodisulfat (30 mg/100ml) versetzt und 9,5 h bei 70°C polymerisiert. Durch Zentrifugation wird das in der Volumenphase synthetisierte Polymer PDADMAC abgetrennt. Durch Behandlung mit 100 mM 6-CF, das an die Aminogruppen von PDADMAC bindet, kann das Vorhandensein von an die negativ geladenen Kapselwände adsorbiertem Polymer und im Inneren der Kapsel befindlichem Polymer nachgewiesen werden.

Aus 9 Schichten bestehende Polyelektrolythüllen ([PSS/PAH]₄PSS) werden auf Humanerythrozyten abgeschieden und die Templatpartikel entfernt. Anschließend wird eine weitere Schicht PAH aufgetragen. Die Kapseln werden zur radikalischen Polymerisation von Acrylsäure zu Polyacrylsäure verwendet. Hierzu wird eine 3%ige Monomerlösung in einer 2%igen Kapselsuspension mit dem Initiator Natriumperoxodisulfat (30 mg/100 ml) versetzt und 9,5 h bei 70°C polymerisiert. Durch Zentrifugation wird die in der Volumenphase synthetisierte Polyacrylsäure entfernt. Nach Zugabe von 100 mM Rhodamin B, das selektiv an anionische Gruppen bindet, kann das Vorhandensein von Polyacrylsäure adsorbiert an die negativ geladenen Kapselwände, aber auch im Inneren der Kapseln nachgewiesen werden. Die Adsorption von Acrylsäure an Kapselwände kann durch Verwendung von Kapseln mit einer äußeren negativen Ladung verhindert werden.

### Beispiel 11 Kolloidale Stabilisierung von mit organischen Lösungsmitteln gefüllten Polyelektrolythüllen

Polyelektrolythüllen in wässriger Lösung werden mit DPPA (unter Zusatz von 5% markiertem DPPC) beschickt. Die wässrige Dispersion wird mit Pentanol, Octanol oder Decan gemischt. Die Proben werden für 2 min bei 17.000 Upm zentrifugiert. Die Mischung trennt sich in zwei Phasen auf, wobei die Hüllen an der Zwischenschicht zwischen der wässrigen und der organischen Phase lokalisiert sind. Die wässrigen Phase wird extrahiert und die Probe dreimal mit dem organischen Lösungsmittel gewaschen.

Durch konfokale Mikroskopie kann nachgewiesen werden, daß die Lipide selbst nach Kontakt mit dem organischen Lösungsmittel an den Hüllen verbleiben und daß im Inneren der Hülle eine wäßrige Phase verkapselt ist. Hierzu werden die Kapseln vor dem Aufbringen der Lipidschicht 1 h in einer 0,1 mM 6-CF-Lösung inkubiert und die Probe nach Aufbringen der Lipide viermal mit Wasser zur Entfernung von überschüssigen Lipiden und 6-CF gewaschen. Ein 12 h nach der Präparation aufgenommenes konfokales Bild zeigt, daß die wässrige 6-CF-Lösung immer noch innerhalb der Hüllen verkapselt ist. Das organische Lösungsmittel zeigt keine Fluoreszenz.

Diese Ergebnisse zeigen, daß die Polyelektrolythüllen zur Verkapselung organischer Lösungsmittel in Wasser und auch umgekehrt zur Verkapselung einer wässrigen Phase im organischen Medium verwendet werden können. Somit können stabile Öl-in-Wasser- und Wasser-in-ÖI-Emulsionen ohne Verwendung oberflächenaktiver Mittel erzeugt werden.

### Beispiel12 Herstellung von Polyelektrolythüllen durch Membranfiltration

### 12.1 Materialien

Als Templatpartikel wurden geladene Polystyrol-Latexteilchen mit einem Durchmesser von 640 nm, die nach der Methode von Furosawa et al. (Colloid-Z. Z. Polym. 250 (1972), 908) hergestellt wurden, teilvernetzte Melaminformaldehydpartikel mit Durchmessern von 3,7 µm und 5 µm sowie mit Glutardialdehyd fixierte humane Erythrozyten verwendet. Als Beschichtungssubstanzen werden Natriumpoly(stryrolsulfat) PSS (MW 70.000), Poly (allylaminhydrochlorid) PAH (MW 8.000 bis 11.000), Poly(diallyldimethylammoniumchlorid) PADMAC (MW 100.000), Chitosan (MW 200.000 bis 300.000), Chitosansulfat (MW 200.000 bis 300.000) und Magnetit-partikel verwendet. Hierzu werden wässrige Lösungen von 1 oder 2 mg/ml PSS, PAH oder PDADMAC in 0,5 M NaCl hergestellt. Chitosansulfat wird als Lösung mit einer Konzentration von 1 mg/ml in 0,5 M NaCl hergestellt. Chitosan wird in einer Konzentration von 1 mg/ml in 0,5 M NaCl unter Zusatz von 0,3% (v/v) Essigsäure aufgelöst.

Es wird die Vakuumpumpe SM 16692 (Sartorius AG, Göttingen, Deutschland) zur Membranfiltration verwendet, mit der ein Vakuum von etwa 100 mbar und ein Überdruck bis zu 3 bar erzeugt werden kann. Zur Vakuumfiltration wird die Polycarbonatfiltrationseinheit SM 16510 (Sartorius) und zur Druckfiltration die Einheit SM 16526 (Sartorius) verwendet.

Es werden Membranfilter mit einem Durchmesser von 47 mm der folgenden Typen verwendet: Sartolon Polyamid SM 25007-047 N (0,2 µm), Sartolon Polyamid SM 25006-047 N (0,45 µm), Celluloseacetat SM 11104-047 N (0,8 µm) und Cellulosenitrat SM 11306-100 N (0,45 µm).

### 12.2 Methoden

Polystyrol- und Melaminformaldehyd-Latexsuspensionen werden in einer Konzentration von 1 bis 30 % (v/v) eingesetzt. Die Konzentration der Erythrozytensuspension sollte etwa 10% (v/v) nicht übersteigen. Das Volumen der im ersten Adsorptionsschritt verwendeten Partikelsuspensionen ist zwischen 10 ml und 50 ml. Die Adsorptionsdauer ist immer 5 min. Danach werden die Partikel mit Wasser gewaschen.

Die Membranfiltration kann als Vakuumfiltration, Druckfiltration und Filtration ohne Druckveränderung durchgeführt werden. Während der Polyelektrolytadsorption wird vorzugsweise ein schwacher Unterdruck in der Inkubationskammer bezüglich der unteren Filtratkammer angelegt, um einen Verlust des Inkubationsmediums und des Polyelektrolyten während der Adsorption zu vermeiden.

Ein Gesichtspunkt bei der Auswahl des Membranfilters ist das Ladungsvorzeichen des zu adsorbierenden Polyelektrolyten. Im Falle von Polykationen (PAH, Chitosan) sind beispielsweise Polyamidfilter geeignet. Im Falle von Polyanionen können Celluloseacetat oder Cellulosenitratfilter verwendet werden. Auf diese Weise kann Verstopfen des Filters durch Polyelektrolytadsorption gering gehalten werden.

Zweckmäßigerweise wird die Filtration unter Bedingungen durchgeführt, bei denen ein Entstehen oder Zusammenbacken des Filterkuchens vermieden bzw. begrenzt wird. Die Adhäsion von PS und MF-Latexpartikel mit einer äußeren Schicht von PSS oder PAH an die Filteroberflächen ebenso wie deren Aggregations- oder/und Flokkulationsneigung ist gering. Die Filtration kann daher bis zu hohen Teilchenkonzentrationen (20%) ohne Unterbrechung oder Ersetzen des abfiltrierten Suspensionsmediums durch das Waschmedium durchgeführt werden. Im Falle von Erythrozyten muß während der Abscheidung der ersten vier oder fünf Schichten vorsichtig gearbeitet werden, um eine Aggregation zu verhindern. Die Suspension sollte daher nicht auf Konzentrationen von mehr als etwa 5 bis 10% (v/v) eingeengt und die Bildung eines Filterkuchens soweit wie möglich vermieden werden. Nach Abschluß der ersten Adsorptionszyklen nimmt die Aggregationsneigung sowie die Neigung zur Adhäsion an den Filter ab. Eine gegebenenfalls zwischenzeitlich auftretende Flockung während der Zugabe eines Polyelektrolyten kann bei laufendem Prozeß durch Zugabe eines entgegengesetzt geladenen Polyelektrolyten, z.B. des nachfolgenden Polyelektrolyten ohne Schädigung des Produkts wieder aufgelöst bzw. gebrochen werden.

Ähnliche Ergebnisse werden mit dem Chitosan/Chitosansulfat-System erhalten. Eine weitgehend vollständige Unterdrückung der Aggregatbildung kann durch Rühren erreicht werden.

### 12.3 Ergebnisse

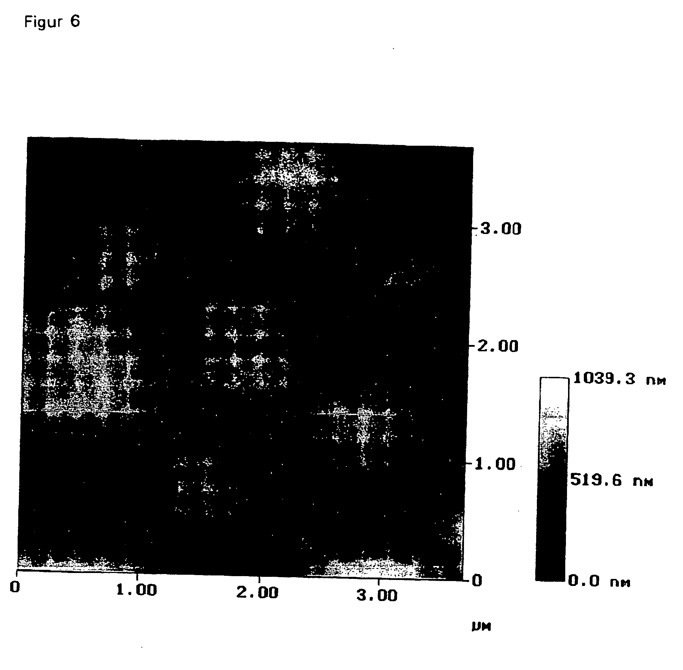

In Figur 6 ist eine AFM-Abbildung von PS-Latexoberflächen nach Aufbringen von 3 PAH/PSS-Schichtpaaren gezeigt. Die Oberflächen sind glatt und es sind keine Polyelektrolytaggregate zu erkennen. Diese Partikel wurden durch Vakuummembranfiltration mit einem Saugdruck von etwa 100 mbar und unter Verwendung von 450 nm Cellulosenitrat-Membranfiltern hergestellt. Ebenso können Polyamidfilter oder abwechselnd negativ geladene Celluloseacetat- und positiv geladene Polyamidfilter verwendet werden.

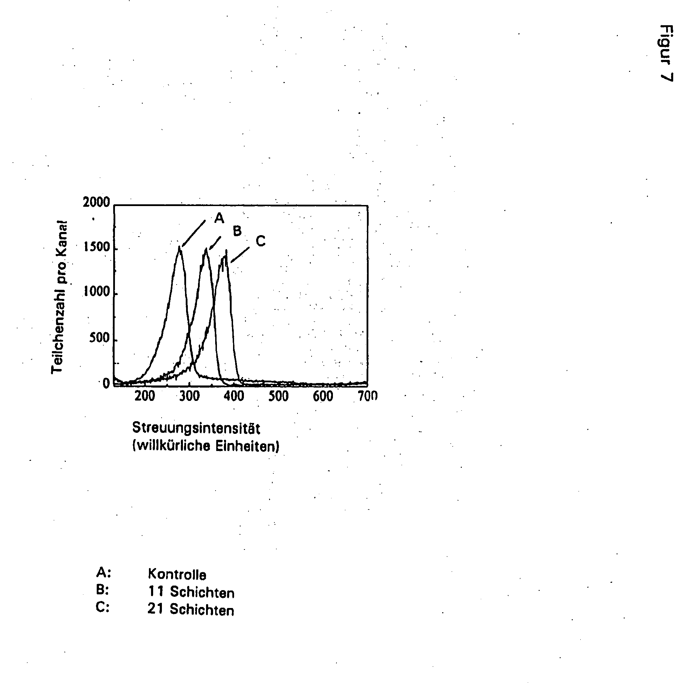

In Figur 7 wird das Wachstum der Hüllendicke an der Oberfläche von 640 nm PS-Latexpartikeln durch Einzelpartikel-Lichtstreuung nach der bei Lichtenfeld et al. (Colloid Surfaces A 104 (1995), 313) beschriebenen Methode bestimmt. Die Streulichtintensität nimmt mit der Teilchengröße zu. Es sind die Ergebnisse von Teilchen mit 11 bzw. 21 PAH/PSS Schichten im Vergleich zu unbeschichteten Teilchen gezeigt.

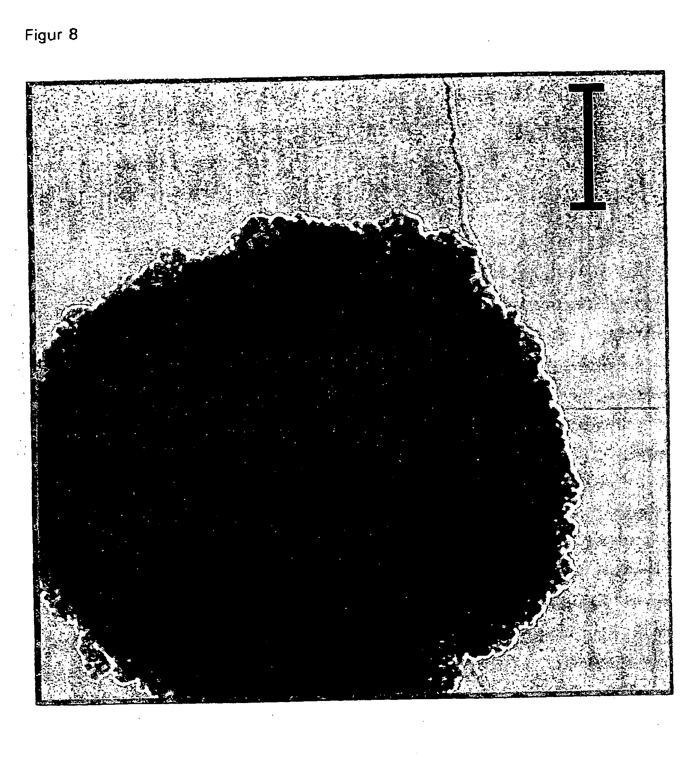

Das TEM-Bild eines mit einer gemischten Schichten aus Polyelektrolyt- und Magnetitteilchen, die durch Präzipitation von Eisen (II)- und Eisen (III)-Salzen in Ammoniumhydroxydlösung und Stabilisierung durch HCl erzeugt wurden (Massart und Cabuil, J. D. Chemie Physique 84 (1987), 967), ist in Figur 8 gezeigt. Zunächst werden zwei PAH/PSS Doppelschichten adsorbiert. Danach werden drei Magnetit/PSS Doppelschichten adsorbiert. Die Filtratlösung war jeweils ungefärbt und klar. Dies bedeutet eine vollständige Adsorption des Magnetits. An der Oberfläche gebildete Magnetitaggregate sind deutlich erkennbar.

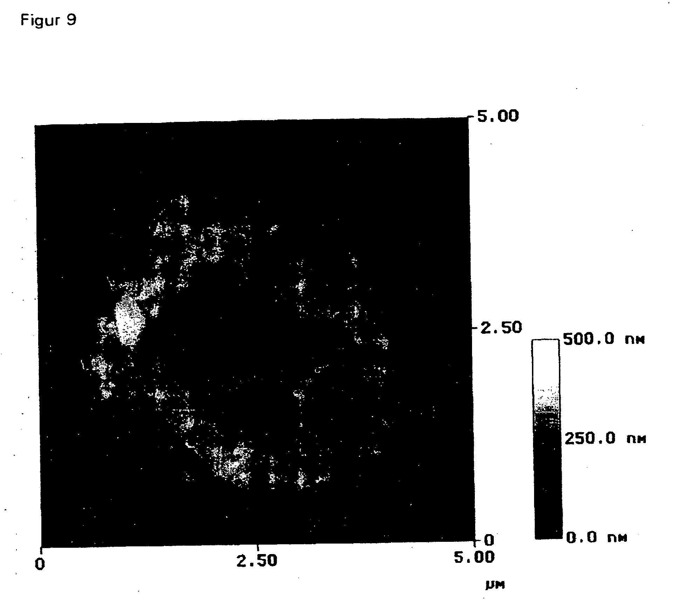

Bei Verwendung von auflösbaren MF-Partikeln als Templat werden ebenfalls gute Ergebnisse bei der Membranfiltration erzielt. Mikrokapseln mit 10 PAH/PSS Schichten auf 3,7 µm MF-Partikeln sind im AFM-Bild von Figur 9 gezeigt. Die Templatpartikel wurden im Citratpuffer pH 1,4 aufgelöst. Die Morphologie der flachen Hülle ist deutlich zu erkennen.

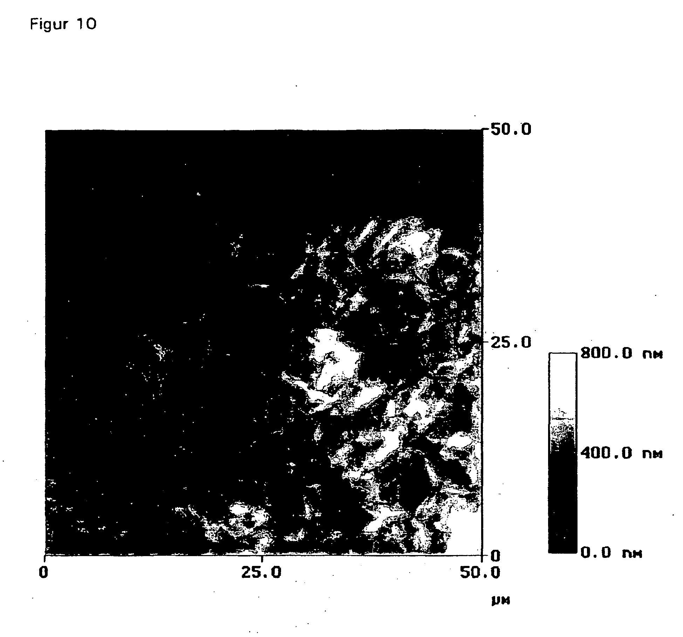

In Figur 10 ist ein AFM-Bild von PAH/PSS Mikrokapseln mit 10 Schichten gezeigt, die an der Oberfläche von Glutaraldehyd fixierten humanen Erythrozyten abgeschieden wurden. Trotz der negativen Ladung der Zellen ist es günstig mit einer PSS-Adsorptionsschicht zu beginnen. Auf diese Weise kann während der ersten Adsorptionsschritte das Ausmaß der Aggregation vermindert werden. Die Filtration sollte unter milden Druckbedingungen (kein oder nur geringer Über- oder Unterdruck) erfolgen, um die Bildung von Filterkuchen zu vermeiden. Ähnliche Vorsichtsmaßnahmen sind bei Verwendung von PDADMAC als Polykation zu beachten. Unter druckfreien Bedingungen werden auch in solchen empfindlichen Systemen hervorragende Ergebnisse erzielt.

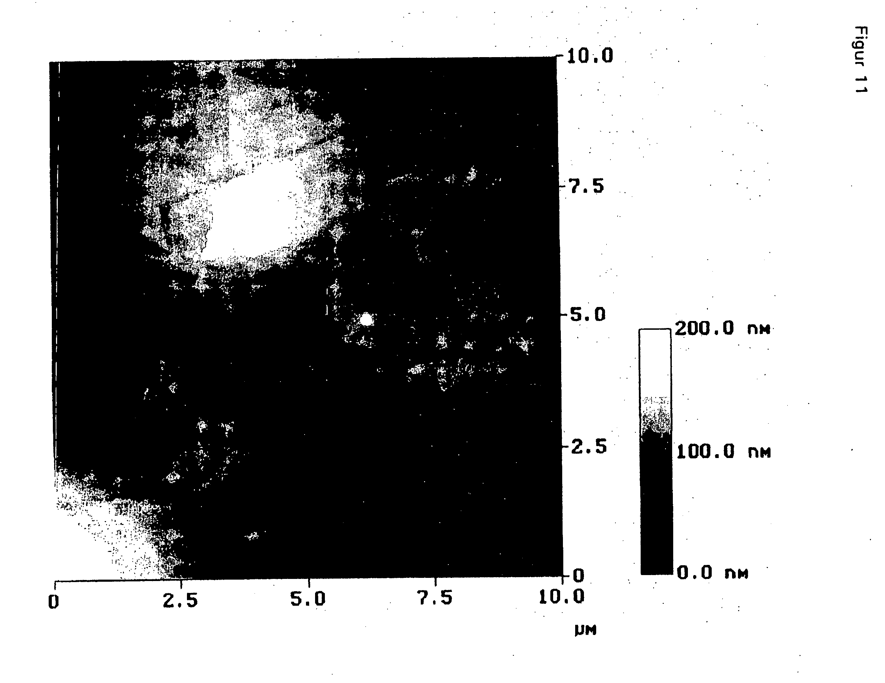

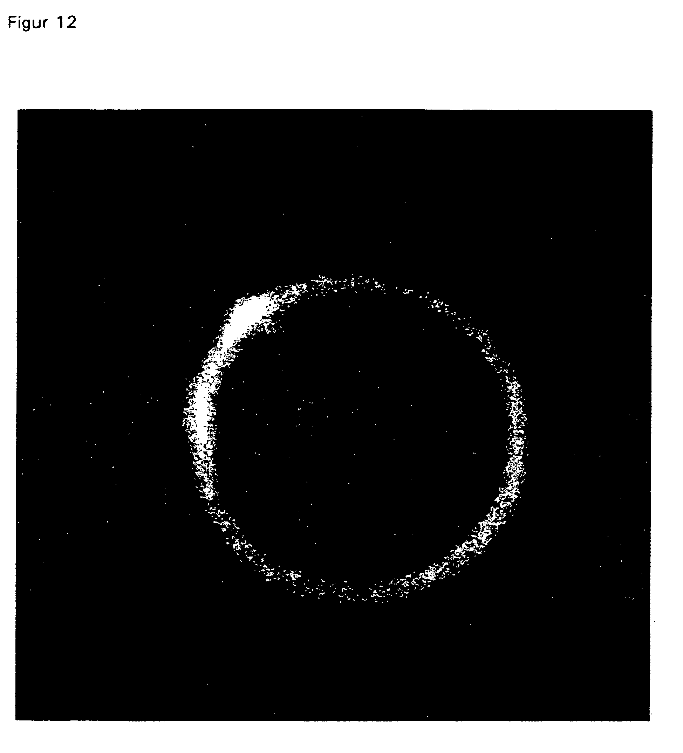

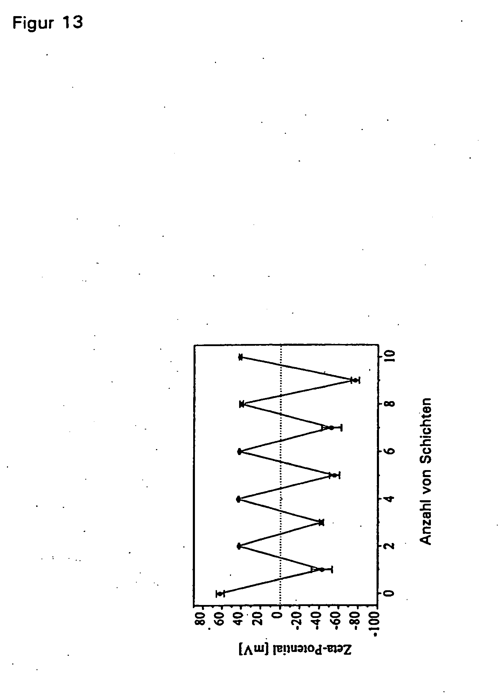

In den Figuren 11 bis 13 sind Ergebnisse dargestellt, die unter Verwendung von Chitosan/Chitosansulfat als Polyelektrolytpaar erhalten wurden. Figur 11 ist ein AFM-Bild von mit 10 Schichten belegten MF-Partikeln nach Auflösung des Kerns. Um die Untersuchung der Hüllmorphologie zu erleichtern wurde mit FITC markiertes PAH als elfte Schicht aufgebracht und die Hülle durch konfokale Laserrastermikroskopie untersucht. Das Ergebnis ist in Figur 12 gezeigt. In Figur 13 sind die Zetapotentiale von schichtweise wachsenden Mikrokapseln angegeben. Ungerade Schichtzahlen entsprechen Chitosansulfat und sind durch negative Zetapotentiale gekennzeichnet. Geradzahlige Schichten entsprechen Chitosan und zeigen positive Zetapotentiale.

### Beispiel 13 Permeabilität von Polyelektrolythüllen

Polyelektrolythüllen wurden durch Beschichtung von 3 µm Melaminformaldehydpartikeln unter Verwendung von Natriumpoly(styrolsulfonat) mit einem Molekulargewicht von 70.000 und Poly(allylaminhydrochlorid) mit einem Molekulargewicht von 50.000 erzeugt. Für die konfokale Fluoreszenzmikroskopie wird ein mit Fluoresceinisothiocyanat markiertes PAH (FITC-PAH) eingesetzt (Sukhorukov, Colloids Surfaces A 137 (1998), 253).

Suspensionen von Melaminformaldehydpartikeln mit 3 µm Durchmesser werden mit 13 Schichten PAH und PSS beschichtet. Dann wird durch 5 min Inkubation in 0,1 NaCl der Kern aufgelöst.

Mittels konfokaler Mikroskopie wird die Permeabilität der Polyelektrolytmikrokapseln untersucht. Hierzu wird zunächst eine Lösung von PAH-FITC (Molekulargewicht 50.000) mit den Hüllen auf eine Endkonzentration von 0,5 mg/ml vermischt. Die Permeabilität der Hüllwände für hochmolekulares PAH-FITC ist so gering, daß nach 20 min Inkubation keine Fluoreszenz im Inneren der Kapseln nachweisbar war. Im Gegensatz dazu kann 6-Carbofluoreszein (6-CF) die Wände der Hüllen ohne weiteres durchdringen.

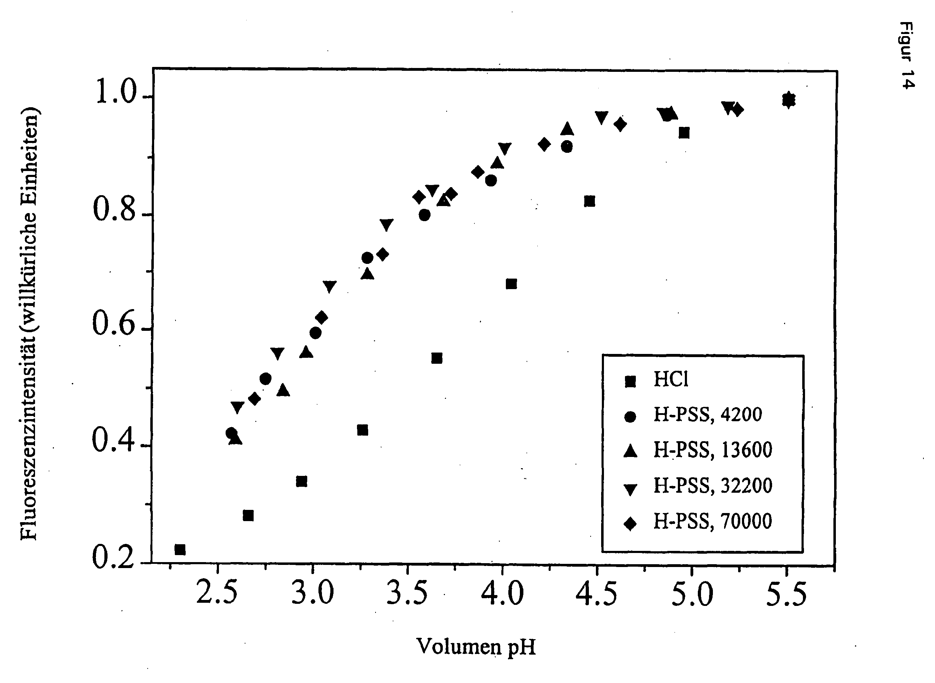

Figur 14 zeigt die Fluoreszenzintensität einer Suspension von 6-CF als Fluoreszenzmarker enthaltenden Polyelektrolytkapseln gegenüber den durch Polystyrolsulfonsäure und HCl titrierten pH-Wert im umgebenden Medium. Die Ergebnisse zeigen, daß Polystyrolsulfonsäure in den jeweils verwendeten Molekulargewichten (70.000-4.200) die Polyelektrolythüllen nicht durchdringen konnte.

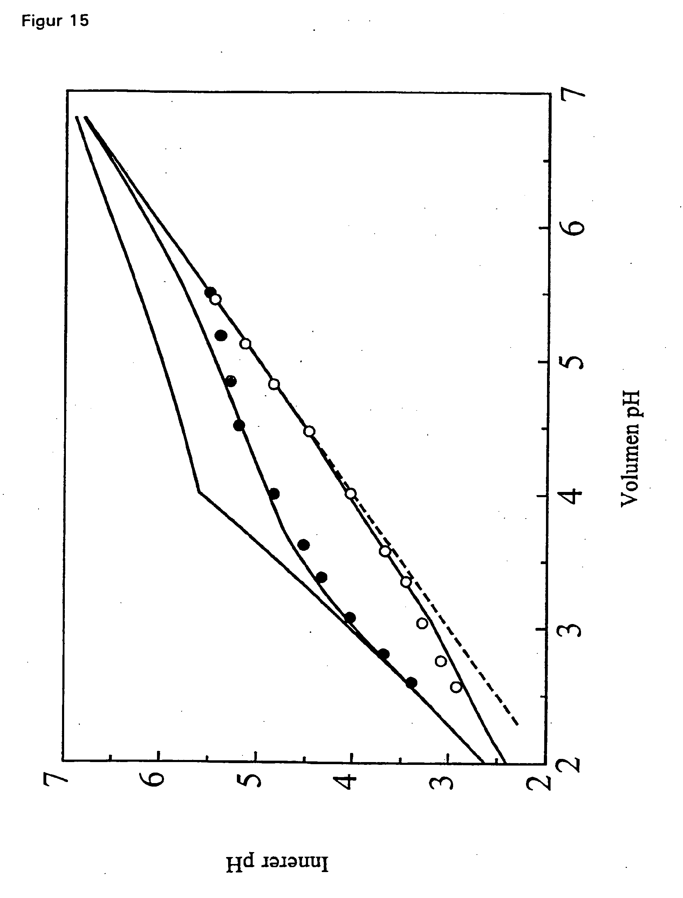

Figur 15 zeigt den pH-Wert im Inneren der Kapseln als Funktion des mit H-PSS (Molekulargewicht 4200) titrierten Volumen pH-Werts in Abwesenheit von Salz und in Gegenwart von 1 mM NaCl. Es ist zu erkennen, daß mindestens innerhalb einer Stunde keine signifikante Diffusion von PSS Polyanionen mit dem jeweils getesteten Molekulargewichten ins Innere der Kapsel stattfindet. Dies führt zum Entstehen eines pH-Gradienten zwischen dem Inneren der Kapsel und dem Volumen. Der pH-Wert im Inneren ist um etwa eine pH-Einheit basischer als der pH-Wert des Volumens. Dies gilt insbesondere für einen Volumen pH-Wert geringer als 5,5.

## Patentansprüche

1. Kapsel mit einer Polyelektrolythülle, die mehrere Polyelektrolytschichten umfasst, und einem Durchmesser bis zu 10 µm,
**dadurch gekennzeichnet,**
**dass** die Hülle Lipide enthält.

2. Kapsel nach Anspruch 1,
**dadurch gekennzeichnet,**
**dass** sie einen Durchmesser ≤ 5 µm aufweist.

3. Kapsel nach Anspruch 1 oder 2, erhältlich durch Aufbringen von Polyelektrolytmolekülen und Lipiden auf ein Templatpartikel.

4. Kapsel nach einem der vorhergehenden Ansprüche,
**dadurch gekennzeichnet,**
**dass** die Hülle abwechselnde Schichten von kationischen und anionischen Polyelektrolyten umfasst.

5. Kapsel nach einem der Ansprüche 1 bis 4,
**dadurch gekennzeichnet,**
**dass** sie das Templatpartikel in ihrem Inneren enthält.

6. Kapsel nach einem der Ansprüche 1 bis 4,
**dadurch gekennzeichnet,**
**dass** das Templatpartikel mindestens teilweise aufgelöst ist.

7. Kapsel nach einem der vorhergehenden Ansprüche,
**dadurch gekennzeichnet,**
**dass** innerhalb der Hülle eine Flüssigphase vorliegt.

8. Kapsel nach einem der vorhergehenden Ansprüche,
**dadurch gekennzeichnet,**
**dass** sie einen Wirkstoff enthält.

9. Kapsel nach einem der vorhergehenden Ansprüche,
**dadurch gekennzeichnet,**
**dass** die Hülle Poren enthält.

10. Kapsel nach Anspruch 9,
**dadurch gekennzeichnet,**
**dass** die Poren den Durchtritt von Substanzen mit einem Molekulargewicht ≤ 4 kD erlauben.

11. Kapsel nach einem der vorhergehenden Ansprüche,
**dadurch gekennzeichnet,**
**dass** die Hülle weiterhin Nanopartikel oder/und Tenside enthält.

12. Kapsel nach einem der vorhergehenden Ansprüche,
**dadurch gekennzeichnet,**
**dass** die Stärke der Hüllwand 2 bis 100 nm beträgt.

13. Kapsel nach einem der vorhergehenden Ansprüche,
**dadurch gekennzeichnet,**
**dass** die Lipide ausgewählt sind aus Phospholipiden, Fettsäuren oder Alkylsulfonsäuren.

14. Kapsel nach Anspruch 13,
**dadurch gekennzeichnet,**
**dass** Lipide ausgewählt sind aus Dipalmityl-Phosphatidinsäure oder Dipalmityl-Phosphatidylcholin.

15. Verfahren zur Modifizierung der Permeabilität von Polyelektrolythüllen,
**dadurch gekennzeichnet,**
**dass** auf der Polyelektrolythülle Lipidschichten abgeschieden werden.

## Claims

1. A capsule with a polyelectrolyte shell which comprises a plurality of polyelectrolyte layers and has a diameter of up to 10 µm,
**characterized in that**
the shell contains lipids.

2. The capsule according to claim 1,
**characterized in that**
it has a diameter of ≤5 µm.

3. The capsule according to claims 1 or 2 which is obtainable by applying polyelectrolyte molecules and lipids to a template particle.

4. The capsule according to any one of the preceding claims,
**characterized in that**
the shell comprises alternating layers of cationic and anionic polyelectrolytes.

5. The capsule according to any one of claims 1 to 4,
**characterized in that**
it contains the template particle in its interior.

6. The capsule according to any one of claims 1 to 4,
**characterized in that**
the template particle is at least partially disintegrated.

7. The capsule according to any one of the preceding claims,
**characterized in that**
a liquid phase is present inside the shell.

8. The capsule according to any one of the preceding claims,
**characterized in that**
it contains an active ingredient.

9. The capsule according to any one of the preceding claims,
**characterized in that**
the shell contains pores.

10. The capsule according to claim 9,
**characterized in that**
the pores permit substances with a molecular weight of ≤4 kD to pass through.

11. The capsule according to any one of the preceding claims,
**characterized in that**
the shell further contains nanoparticles and/or surfactants.

12. The capsule according to any one of the preceding claims,
**characterized in that**
the thickness of the shell wall is 2 to 100 nm.

13. The capsule according to any one of the preceding claims,
**characterized in that**
the lipids are selected from phospholipids, fatty acids or alkyl sulfonic acids.

14. The capsule according to claim 13,
**characterized in that**
the lipids are selected from dipalmitoyl phosphatidic acid or dipalmitoyl phosphatidylcholine.

15. A method for modifying the permeability of polyelectrolyte shells,
**characterized in that**
lipid layers are deposited on the polyelectrolyte shell.

## Revendications

1. Capsule comportant une enveloppe de poly-électrolyte qui comprend plusieurs couches de poly-électrolytes et un diamètre allant jusqu'à 10 µm, **caractérisée en ce que** l'enveloppe contient des lipides.

2. Capsule selon la revendication 1, **caractérisée en ce qu'**elle présente un diamètre ≤ 5 µm.

3. Capsule selon la revendication 1 ou 2, pouvant être obtenue par l'application de molécules de poly-électrolytes et de lipides sur une particule matrice.

4. Capsule selon l'une des revendications précédentes, **caractérisée en ce que** l'enveloppe comprend des couches alternées de poly-électrolytes cationiques et anioniques.

5. Capsule selon l'une des revendications 1 à 4, **caractérisée en ce qu'**elle contient la particule matrice à l'intérieur de celle-ci.

6. Capsule selon l'une des revendications 1 à 4, **caractérisée en ce que** la particule matrice est dissoute au moins partiellement.

7. Capsule selon l'une des revendications précédentes, **caractérisée en ce qu'**à l'intérieur de l'enveloppe se trouve une phase liquide.

8. Capsule selon l'une des revendications précédentes, **caractérisée en ce qu'**elle contient une substance active.

9. Capsule selon l'une des revendications précédentes, **caractérisée en ce que** l'enveloppe contient des pores.

10. Capsule selon la revendication 9, **caractérisée en ce que** les pores permettent la pénétration de substances présentant un poids moléculaire ≤ 4 kD.

11. Capsule selon l'une des revendications précédentes, **caractérisée en ce que** l'enveloppe contient en outre des nanoparticules ou/et des tensioactifs.

12. Capsule selon l'une des revendications précédentes, **caractérisée en ce que** l'épaisseur de la paroi d'enveloppe est de 2 à 100 nm.

13. Capsule selon l'une des revendications précédentes, **caractérisée en ce que** les lipides sont choisis parmi les phospholipides, les acides gras ou les acides alkylsulfoniques.

14. Capsule selon la revendication 13, **caractérisée en ce que** les lipides sont choisis parmi l'acide dipalmityl-phosphatidique ou la dipalmityl-phosphatidylcholine.

15. Procédé de modification de la perméabilité d'enveloppes de poly-électrolytes, **caractérisé en ce que** des couches lipidiques sont déposées sur l'enveloppe de poly-électrolytes.
